# EUROPEAN PATENT SPECIFICATION

(11) **EP 3 429 518 B1**
(45) Date of publication and mention of the grant of the patent: **26.04.2023**
(21) Application number: 17713760.1
(22) Date of filing: 16.03.2017
(51) Int. Cl.: A61F 9/00, A61K 9/00, A61K 47/32

(54) **OPHTHALMIC DELIVERY DEVICE**
VORRICHTUNG ZUR OPHTHALMISCHEN VERABREICHUNG
DISPOSITIF D'ADMINISTRATION OPHTALMIQUE

(30) Priority: 16.03.2016 US 201662309350 P
(43) Date of publication of application: 23.01.2019
(73) Proprietor: Oxular Limited, Oxford OX4 4GA (GB)
(72) Inventor: BLEY, Robert Steven, Menlo Park California 94025 (US); CONSTON, Stanley R, San Carlos California 94070 (US); NGUYEN, Tien T, Daly City California 94014 (US); YAMAMOTO, Ronald, San Francisco California 94117 (US); HOWARTH, Brad Michael, London N1 0GL (GB); STOPS, Adam Jonathan Frederick, Cambridge CB4 1RL (GB); BARNETT, Ricky, St Albans Hertfordshire AL4 9UR (GB)
(74) Representative: Bassil, Nicholas Charles
(86) International application number: PCT/GB2017/050730
(87) International publication number: WO 2017/158365

(56) References cited:
- WO-A1-2013/028936
- WO-A1-2013/151904
- WO-A1-2016/042162
- WO-A2-2016/042163
- US-A1- 2011 238 075

## Description

### Background of Invention:

Due to the unique anatomy and physiology of the eye, multiple barriers exist that prevent significant transport of drugs to ocular tissues. The blood vessels of the eye have restricted permeability due to the blood-ocular barriers that regulate intraocular fluid. Due to these blood-ocular barriers, systemically administered drugs do not reach significant concentration in ocular tissues. Drugs in topical drops administered to the corneal surface are mostly washed out by tears into the naso-lacrimal duct. While in the tear film, drugs have limited time to penetrate the cornea to reach the intraocular space. Some drugs may be delivered to the front, anterior portion of the eye by drops, but reaching significant therapeutic concentrations in the posterior portion of the eye and the retina is generally not achieved with topical methods of administration.

Many diseases that result in visual loss involve the posterior retina where color vision and reading occur. To treat the posterior portion of the eye and the posterior retina typically drugs are injected into the eye. Sub-conjunctival injections are used to place a drug depot under the outer layer of the eye, however the very high lymphatic flow in the conjunctiva leads to rapid transport of the drug away from the eye. Sub-conjunctival injections are typically not effective to achieving high drug levels in the posterior portion of the eye.

Sub-Tenon's injections are sometimes used to place the drug under the conjunctiva and Tenon's capsule of the eye in a more posterior location to deliver drug to the posterior region of the eye. Sub-Tenon's injections have been demonstrated to be useful for the administration of steroids, however many drugs do not achieve significant drug levels in the retinal tissues from sub-Tenon's injection. The tip of the injection needle is placed deep into the posterior shell of the eye where the tip of the needle cannot be directly observed. The technique requires experience and careful technique to avoid physical injury to the eye or misplacement of drug.

Intravitreal injections are given to place drug directly into the vitreous chamber, and typically require a smaller quantity of drug as compared to sub-Tenon's injections. The half-life of the drug is limited due to the fluid in the vitreous which continuously moves forward toward the anterior chamber. This vitreous flow washes out the drug over time and contacts the drug to other tissues of the eye in the flow path. Intravitreally administered drugs such as steroids are associated with complications of cataract progression due to drug exposure to the lens and increased intraocular pressure from drug exposure to the trabecular meshwork during anterior flow from the vitreous chamber.

The suprachoroidal space between the choroid and sclera and the supraciliary space between the ciliary body and sclera are more difficult to locate but also can be used for the injection of drugs.

Unlike intravitreal injections, the fluid in the suprachoroidal space and supraciliary space flows posteriorly. This flow may assist drugs injected into the suprachoroidal space or the supraciliary space to reach the posterior tissues and posterior retina. Small drug particle sizes are ideal for migration in the suprachoroidal space or supraciliary space, however small drug particles release drug at a much faster rate thereby reducing the longevity of the drug treatment.

One potential problem with all injections of drug into the eye beneath the sclera is increased intraocular pressure (IOP) caused by the additional volume introduced into the eye. The increased IOP may cause pain and potential damage to the optic nerve. For highly active drugs a small injection volume may be used without significant acute IOP increase, for example 0.05 ml of anti-VEGF drugs. However, for larger volumes such as 0.1 ml with steroids, IOP increase may be significant and may cause an acute period of pain and loss of vision.

WO 2013/028936 A1 describes a delivery device comprising an elongated body with a hollow needle at a distal end, a reservoir, a plunger and a distal seal.

### Summary of the Invention:

The present invention provides a delivery device comprising an elongated body with a needle with a lumen at a distal end; a first reservoir for a volume of gas to be delivered through the needle; a second reservoir for an active agent containing composition to be delivered through the needle; a gas flow path from the first reservoir to the needle lumen; a first plunger with a force element configured to pressurise the volume of gas in the first reservoir prior to or simultaneous with contact of the distal end of the device to a tissue surface; a second plunger or a push rod configured to provide a delivery force from the force element to the active agent containing composition; a distal element attached to the distal end of the device configured to seal the needle lumen from delivery of the gas under the delivery force; and a mechanism configured to provide a sequential delivery process for the volume of gas and the active agent containing composition; wherein: the distal element comprises a tissue interface and a distal seal, and the distal seal is penetrable by a distal tip of the needle by the application of pressure on a tissue surface with the distal end of the device; the distal element being penetrated is configured to be slidable on the needle to allow advancement of the needle into tissue; the distal seal being penetrated is configured to open a path for flow or delivery of the gas from the first reservoir through the gas flow path to the distal end of the needle; and the active agent containing composition from the second reservoir is delivered through the needle by the second plunger or push rod and force element after all or a portion of the volume of gas has been delivered through the needle.

The force element of the delivery device may comprise a first force element to apply a delivery force to compress the volume of gas and a second force element to apply a delivery force to the active agent containing composition. The gas flow path may comprise the needle lumen, a portion of the needle lumen or a separate lumen. The gas flow path may also comprise a sleeve coaxial to the needle and a hole in a shaft of the needle within the sleeve.

The delivery of the active agent containing composition can be manually triggered by the user after delivery of at least a portion of the volume of gas. The delivery of the active agent containing composition may be automatically triggered by a mechanism after delivery of at least a portion of the volume of gas. The mechanism may be configured to sequentially provide a delivery force to the volume of gas and the active agent containing composition. The mechanism may be configured to block the delivery of the active agent containing composition until delivery of at least a portion of the volume of gas. The mechanism may comprise a linkage between the first force element acting on the volume of gas and the second force element acting on the active agent containing composition. The mechanism may comprise a linkage between the plunger acting on the volume of gas and the plunger or push rod acting on the active agent containing composition.

The needle of the delivery device may comprise a curved distal tip to direct the active agent containing composition at an angle from the long axis of the needle. The needle may comprise an inner deflecting element in the lumen of the needle at the needle bevel to direct the active agent containing composition at an angle from the long axis of the needle. The needle may comprise a deflecting element in the lumen of the needle at the needle bevel to fragment the active agent containing composition prior to delivery of the material for administration from the needle distal tip. The deflecting element may have a width of 0.20% to 50% of the needle inner diameter. The deflecting element may have a height of 0.25% to 50% of the needle inner diameter.

The delivery device may additionally comprise a further force element between the body of the device and the distal element configured to provide a forward directed force on the distal element during penetration of the distal seal by the distal tip of the needle. The tissue interface may comprise an elastomer with a hardness of 10 to 30 Shore A. The forward directed force may be in the range of 40 to 82 gram force.

The delivery device may further comprise an active agent containing composition wherein the active agent containing composition is a solid or semisolid. The reservoir may be within the lumen of the needle. The reservoir may be within both the lumen of the needle and an extension of the needle into the body of the device.

The delivery device may additionally comprise a collapsible element between the body of the device and the distal element configured to prevent distal movement of the distal element due to the delivery force. The collapsible element may comprise elongated struts. The collapsible element may comprise nitinol or polyimide.

The delivery device may additionally comprise a collapsible element between the body of the device and the distal element configured to provide a forward directed force on the distal element during penetration of the distal seal by the distal tip of the needle. The collapsible element may be configured to provide a constant force after an initial force wherein the initial force is applied during the first 0.5 mm of travel of the distal element proximally along the needle. The tissue interface and distal seal may be mounted on a tubular distal housing.

The first and/or second force element of the delivery device may be a spring. The first and/or second force element of the delivery device may be a pressurized gas. The delivery force may be activated by a mechanism to compress the first and/or second force element from the exterior of the device. The first and/or second force element may be constrained prior to use and the delivery force is activated by mechanically releasing the constrained force element.

The delivery device may be for administration of an active agent containing composition to the suprachoroidal space or supraciliary space with an effective full needle length of 1 to 4 mm. The delivery device may be for administration of an active agent containing composition to the subconjunctival space with an effective full needle length of 0.35 to 2 mm.

In keeping with the foregoing discussion, the present disclosure describes a device is designed for the delivery or implantation of a solid material comprising an active agent shaped as an elongated body. The delivery device comprises an elongated barrel with a hollow needle at the distal end, where the lumen of the needle serves as the reservoir for the elongated body and a plunger or push rod with a force element such as a spring or gas reservoir that provides a delivery force to the elongated body. The elongated body is sized with a diameter less than or equal to the inner diameter of the needle lumen. As described herein, the delivery device also comprises a distal element comprising a tissue interface with a distal seal secured to the distal end of the delivery device thereby sealing the needle lumen during application of the delivery force. The distal seal is penetrable by the distal tip of the needle by the application of pressure on the tissue surface with the distal end of the delivery device and the penetrated distal element becomes slidable on the needle to allow advancement of the needle into tissue. Penetration of the distal seal opens a path for delivery of the material for administration from the distal end of the needle. The delivery device with a force element is activated prior to or simultaneous with penetration of the distal seal by the needle and advancement of the needle tip into tissues, thereby enabling simple one-handed operation of the delivery device to administer the active agent containing composition shaped as an elongated body to an eye. As described herein, the distal tip of the needle is curved or incorporates an inner deflecting element in the needle lumen to direct the solid element at an angle from the long axis of the needle during delivery. As described herein, the active agent containing composition is directed at an angle from the long axis of the needle during delivery in a posterior direction. As described herein, the active agent containing composition shaped as an elongated body is preloaded in the delivery device, whereby the delivery device serves as the storage container for the active agent containing composition prior to use. As described herein, the preloaded device is sterilized for use after placement and sealing of the elongated body in the delivery device.

The present disclosure describes a device designed for the administration of a semisolid material comprising an active agent. The delivery device comprises an elongated barrel with a hollow needle at the distal end, and a reservoir in the elongated body containing the semisolid material and a plunger or push rod with a force element such as a spring or gas reservoir that provides a delivery force on the semisolid material. As described herein, the semisolid material is designed to flow under delivery pressure provided by the force element and plunger or push rod, resulting in flow through the distal needle into tissues, but the active agent containing composition sets as a semisolid to remain at the administration site during and immediately after administration. As described herein, the semisolid comprises particles or microparticles comprising an active agent, whereby the dissolution of the semisolid at the administration site over time allows natural physiological flow within a tissue space such as the suprachoroidal or supraciliary space to direct the particles posteriorly. As described herein, the delivery device also comprises a distal element comprising a tissue interface with a distal seal secured to the distal end of the delivery device thereby sealing the needle lumen during application of the delivery force. The distal seal is penetrable by the distal tip of the needle by the application of pressure on the tissue surface with the distal end of the delivery device and the penetrated distal element becomes slidable on the needle to allow advancement of the needle into tissue. Penetration of the distal seal opens a path for delivery of the material for administration from the distal end of the needle. The force element of the delivery device is activated prior to or simultaneous with penetration of the distal seal by the needle and advancement of the needle tip into tissues, thereby enabling simple one-handed operation of the delivery device to administer the semisolid composition to an eye. As described herein, the distal tip of the needle is curved or incorporates an inner deflecting element in the needle lumen to direct the semisolid material at an angle from the long axis of the needle during delivery. As described herein, the semisolid material is directed at an angle from the long axis of the needle during delivery in a posterior direction. As described herein, the semisolid material is preloaded in the delivery device, whereby the delivery device serves as the storage container for the active agent containing composition prior to use. As described herein, the semisolid material is preloaded in the delivery device in a dry form whereby the delivery device serves as the storage container for the active agent containing composition and the semisolid material is hydrated in the delivery device prior to use. As described herein, the preloaded device is sterilized for use after placement and sealing of the semisolid material in the delivery device.

The present disclosure describes a device designed for the delivery of a controlled amount of gas to facilitate the administration of a material comprising an active agent to the suprachoroidal space or supraciliary space. The delivery device comprises a gas reservoir and a flow path between the reservoir and the needle lumen, where the gas is introduced to the needle lumen proximal to the distal seal. The gas is pressurized with a force element such as a spring, compressed bladder or expanding element. The gas reservoir is activated prior to or simultaneous with penetration of the distal seal by the needle and advancement of the needle tip into tissues. The flow path for the gas may comprise the needle lumen, a portion of the needle lumen or a separate lumen. As described herein, the flow path comprises an outer sleeve that is coaxial to the needle and the needle has at least one hole along the shaft of the needle within the coaxial outer sleeve. The gas flows from the gas reservoir through the outer sleeve and into the needle lumen through the hole and into the eye from the distal tip of the needle. The resultant gas flow path bypasses the active agent containing composition in the proximal end of the needle to provide an unrestricted flow path to the distal end of the needle. Subsequent to the delivery of the gas, the active agent containing composition is administered through the needle to the suprachoroidal space or supraciliary space. The delivery of the active agent containing composition may be triggered manually after gas delivery. The device incorporates a mechanism to administer the active agent containing composition after all or a portion of the gas has been delivered through the needle to provide a sequential delivery process for the gas and active agent containing composition. As described herein, the delivery device has a first reservoir for the delivery of gas, a second reservoir for the delivery of the active agent containing composition and a force element to deliver both the gas and the active agent containing composition. As described herein, the delivery device has a first reservoir for the delivery of gas, a second reservoir for the delivery of the active agent containing composition, a force element to deliver both the gas and the active agent containing composition and a flow path for the gas delivery comprising an outer sleeve that is coaxial to the needle. As described herein, the delivery device has a first reservoir for the delivery of gas, a second reservoir for the delivery of the active agent containing composition, a force element to deliver both the gas and the active agent containing composition, a flow path for the gas delivery comprising an outer sleeve that is coaxial to the needle and at least one hole in the shaft of the needle in the region of the outer sleeve for gas flow to the lumen of the needle. As described herein, the delivery device has a first reservoir and force element for the delivery of gas and a second reservoir and force element for the delivery of the active agent containing composition. As described herein, the delivery device has a first reservoir and force element for the delivery of gas and a second reservoir and force element for the delivery of the active agent containing composition and a flow path for the gas delivery comprising an outer sleeve that is coaxial to the needle. As described herein, the delivery device has a first reservoir and force element for the delivery of gas and a second reservoir and force element for the delivery of the active agent containing composition, a flow path for the gas delivery comprising a hollow member that is external to the needle and the needle has at least one hole in the shaft in communication with the external hollow member for gas flow into the needle lumen. As described herein, the delivery device has a first reservoir and force element for the delivery of gas and a second reservoir and force element for the delivery of the active agent containing composition, a flow path for the gas delivery comprising an outer sleeve that is coaxial to the needle and the needle has at least one hole in the shaft in the region of the outer sleeve for gas flow into the needle lumen. As described herein, the device comprises a gas reservoir, a flow path for the delivery of gas, a second reservoir for an active agent containing composition and a force element for the delivery of the active agent containing composition through a needle with an inner deflecting element.

The present disclosure describes an active agent containing composition for delivery to the suprachoroidal space, supraciliary space or other tissue spaces of the eye such as the vitreous cavity, subconjunctival space, sub-Tenon's space and sub-retinal space. The active agent containing composition is in the form of a semisolid material whereby the material flows under pressure in a delivery device, but sets into a semisolid once exiting the distal tip of the needle to remain at the administration site. As described herein, the active agent containing composition comprises a plurality of drug-containing particles and at least one excipient to form the drug particles into a semisolid. As described herein, the active agent containing composition comprises a plurality of drug-containing particles and at least one excipient to form the drug particles into a slurry or suspension. As described herein, the excipient comprises a substance that undergoes dissolution in the physiological conditions of the tissue space after delivery to allow the drug-containing particles to disperse and migrate in the tissue space. As described herein, the active agent containing composition is in a dry form that is rehydrated prior to administration.

As described herein, the drug-containing particles are in the form of drug crystals. As described herein, the drug-containing particles are in the form of microspheres. The microspheres may have a coating and/or be made with a biodegradable or bioerodible polymer component to modify the rate of drug release. Similarly, the excipient material may be chosen to control the rate of drug release.

These and other instances of the disclosure will be made apparent from consideration of the following detailed description in conjunction with the accompanying drawing figures.

### Brief Description of the Drawings:

FIG. 1 depicts a solid material delivery device.
FIG. 2 depicts a distal tip of a solid material delivery device.
FIG. 3 depicts a semisolid material delivery device.
FIG. 4 depicts a distal tip of a semisolid material delivery device.
FIG. 5 depicts a distal tip of a delivery device with a collapsible element.
FIG. 6 depicts magnified detail of a distal tip of a delivery device with a collapsible element.
FIG. 7 depicts a distal tip of a delivery device in an uncollapsed state.
FIG. 8 depicts a delivery device in a collapsed state.
FIG. 9 depicts a delivery device needle with a curved distal tip to direct the delivered material at an angle from the long axis of the needle.
FIG. 10 depicts a delivery device needle with an inner deflecting element in the needle lumen at the distal tip to direct the delivered material at an angle from the long axis of the needle.
FIG. 11 depicts a delivery device needle with a protrusion in the needle lumen at the distal tip to fragment a solid element delivered through the needle.
FIG. 12 depicts a delivery device expelling a semisolid material for administration with a plurality of drug-containing particles.
FIG. 13A and 13B depict a formed solid material for administration with a plurality of drug-containing particles.
FIG. 14 is a graph of test results of the tissue interface minimum sealing force.
FIG. 15A depicts a delivery device with a gas delivery mechanism comprising a gas reservoir, a force element to pressurize the gas and gas flow path to the needle lumen.
FIG. 15B depicts Fig. 15A further comprising a coaxial outer sleeve and a needle with a hole along the shaft of the needle within the outer sleeve to allow for the passage of a gas from the gas reservoir into the needle lumen.
FIG. 16 depicts a delivery device with a gas delivery mechanism with a manually triggered delivery mechanism for a solid active agent containing composition.
FIG. 17A through 17F depicts a delivery device with a gas reservoir coaxial to a push rod for delivery of an active agent containing composition with a mechanism to provide sequential gas delivery and active agent containing composition delivery.
FIG. 18A through 18E depicts a delivery device with a gas reservoir, a gas reservoir force element, a reservoir for the active agent containing composition and a force element for the active agent containing composition with a mechanism to provide sequential gas delivery and active agent containing composition delivery.
FIG. 19 depicts a delivery device with a gas delivery mechanism incorporating a vent hole.

### Description of the Disclosure:

To place an active agent containing composition for administration into a tissue space of an eye, the composition is placed in a delivery device. In the present disclosure, the terms delivery is used to describe the administration or implantation of a variety of materials by the disclosure to various tissues of an eye. The device comprises an elongated body with a hollow needle at the distal end, a slidable plunger or push rod at the proximal end, and a reservoir for the material to be administered residing between the distal tip of the needle and the plunger or push rod. In the present disclosure, the term plunger is used to indicate an elongated mechanical element with a seal to provide an interference fit with the inner surface of a reservoir. With some materials for administration such as a solid or sufficiently cohesive semi-solid material for administrations, a seal may not be required for delivery of the material for administration and either a plunger or a push rod without a seal may be used to apply a delivery force to the material for administration. The reservoir is configured to receive a material for administration to be delivered through the lumen of the needle. In particular, the material for administration is a semisolid or solid active agent containing composition. The active agent may be a substance that provides a therapeutic or diagnostic effect for treatment of an eye. The active agent may comprise a drug, a diagnostic agent, gene therapy agents, therapeutic cells or means for physical tissue repair.

The plunger or push rod acts to push the material for administration through the needle to the desired tissue location. A plunger or push rod with a force element is configured to provide a delivery force to the material for administration. The force element may comprise a spring mechanically coupled to a plunger or push rod. The force element may be at least partially located within the elongated body of the device. The plunger or push rod is mechanically coupled to a source of force such as a spring or pressurized gas reservoir such that a delivery force is applied to the material for administration within the device after the material for administration is placed in the reservoir and prior to insertion of the distal end of the needle into ocular tissues to deliver the material for administration into an eye. Secured to the distal end of the needle is a distal element comprising a distal seal, which also acts as a tissue interface. The distal element is moveably secured to the distal tip of the needle where it serves to close off the distal end of the needle to close or block the path of the material for administration from the needle tip. As described herein, the distal element has a lumen to fit over the outer diameter of the needle. As described herein, the distal element is secured to the distal tip of the needle through other means. The distal seal of the distal element is distal to the tip or lumen of the needle and is configured to be penetrated by the needle as the distal tip of the device is placed on the surface of the eye and the needle is advanced into the eye by the user. The needle penetrates the distal seal and is inserted into ocular tissue, thereby opening a flow path or path of delivery of the material for administration from the reservoir, through the needle and into the eye. The resulting self-actuating delivery mechanism insures opening of the delivery path for the material for administration immediately when the needle is placed in tissue, regardless of the orientation and speed of needle insertion.

As described herein, the distal element comprises a tissue interface and distal seal mounted on a tubular distal housing. The tubular distal housing is fit to the exterior of the needle and may be sealed to the surface of the needle at some point along its length. As described herein the housing may be sealed by means of an elastomeric element which is compressed between the housing and the needle. The elastomeric element may therefore be annular. As described herein, the elastomeric element may be compressed between the housing and the body of the device. The elastomeric element may reside at or near the proximal end of the housing. As described herein the elastomeric element serves as a seal between the housing and the needle. As described herein the elastomeric element serves as a frictional element or component which limits the housing travel in the proximal direction to thereby apply a force against the tissue surface by the tissue interface as the needle penetrates the tissues. As described herein, the distal element comprises a tissue interface and a distal seal and is slidably attached to the exterior of the needle without a distal housing.

The distal element, which comprises a tissue interface with a distal seal, or a tissue interface with a distal seal and an attached housing, is attached to the distal tip of the needle but is not freely movable or slidable proximally from the end of the needle due to the closed distal seal. After the material for administration is loaded into the device and the device is activated for use, the material comes under pressure from the source of force but cannot move through the distal seal. The tissue interface is placed on the surface of the eye and the device is manually advanced, thereby forcing the needle through the distal seal and then through the external surface of the eye into underlying tissues. The distal element, after penetration of the distal seal, becomes slidable in the proximal direction from the end of the needle to retain the tissue interface on the surface of the eye during advancement of the needle into tissue. When the distal tip of the needle penetrates through the distal seal, the source of force immediately allows for expression of the material for administration from the needle tip and into the tissues.

As described herein the tissue interface and distal seal is secured to a housing disposed about the needle. The housing may be comprised of a cylindrical element which is secured to the distal end of the body of the device at the proximal end of the housing. The housing may contain collapsible, distortable or deformable elements which allow the distal end of the housing to retract slidably along the needle, which in turn allows the needle tip to penetrate the distal seal. As described herein, the distal element is secured to the distal tip of the needle through other means.

As described herein, the device comprises an elongated barrel with a hollow needle at the distal end and a slidable plunger or push rod at the proximal end. The lumen of the needle serves as the reservoir for the material for administration shaped as an elongated body with a diameter less than or equal to the inner lumen of the needle. The plunger or push rod can be actuated either manually or with a force element such as a spring or pressurized gas source, to push the plunger or push rod and eject the material for administration into the tissue space when the distal tip of the device reaches the space.

As described herein, the device comprises an elongated barrel with a hollow needle at the distal end, a slidable plunger or push rod at the proximal end and a reservoir for the material to be administered residing between the needle and the plunger or push rod. The reservoir of the device contains a liquid or semisolid composition as the material for administration. The plunger or push rod can be actuated either manually or with a force element such as a spring or pressurized gas source, to push the plunger or push rod and eject the material for administration into the tissue space when the distal tip of the device reaches the space.

Operation of the device mechanism opens the path for the material for administration to move out from the tip of the needle immediately upon penetration through the distal seal which occurs just prior to the entry of the needle into the target tissue. Since the material for administration is under pressure prior to or simultaneous with penetration of the distal seal by the needle tip, the delivery is triggered solely by placement and subsequent advancement of the needle through the tissue interface. This allows precise and automatic control of the timing of the delivery action solely due to the needle tip entering the target tissue. The resultant self-actuated mechanism obviates the need for a separate control mechanism, for example a valve or trigger on the body of the delivery device, and hence allows for administration of the material for administration without the need for special positioning of the fingers or the use of the second hand. The device thereby enables an administration to be performed with a single hand, allowing the other hand of the physician to stabilize the eye or perform other actions to facilitate administration. The self-actuating delivery mechanism also eliminates the need for the user to determine when to begin administration which is especially useful when the target tissue space is difficult to locate due to small target size, lack of visualization and anatomic variability such as the suprachoroidal space or supraciliary space.

The device allows precise control of the position of the needle by the user during use. The needle is fixed to the body of the device to allow direct control of the distal tip of the needle when the device is held by the body. Since the delivery force is provided by the force element, the plunger or push rod of the device does not have to be held, triggered or actuated by the hand holding the device, allowing the device to be held and used in a natural, highly controllable position such as with a writing instrument or scalpel. Generally, the needle is arranged parallel to the elongated body or barrel of the device.

Once the flow path from the distal end of the needle is opened by penetration of the distal seal and insertion into the eye, the material for administration cannot flow or move into the eye until a space to accept the material for administration is reached by the distal end of the needle. Scleral tissue in particular is very resilient and effectively seals the needle tip during passage of the needle tip to the suprachoroidal or supraciliary space, hence the unique properties of the sclera do not allow for the material for administration to enter the sclera. Once an underlying space such as the suprachoroidal space or the supraciliary space is reached by the needle tip, the material for administration is able to flow or move out of the needle and be delivered to the space. By this mechanism the material for administration is directed to a location that can accept the material for administration at the distal tip of the needle. The delivery of the material for administration may be further directed by the tissue interface. The tissue interface may optionally apply a force to the surface of the eye to aid sealing of the needle tract at the surface of the eye. With an appropriate needle length and orientation, the device may be used to deliver into the sub-conjunctival space, sub-Tenon's space, suprachoroidal space, supraciliary space, sub-retinal space, the vitreous cavity, or the anterior chamber.

As described herein, the material for administration is a solid or semisolid material. The material for administration may be loaded into the distal end of the needle lumen. The needle may extend proximally in the body of the device to provide an extended length for the material for administration. A plunger or push rod is inserted into the proximal end of the needle and the distal end of the plunger or push rod is put into contact with the proximal end of the material for administration. After placement of the plunger or push rod, a force element such as a compression spring acting on the plunger or push rod provides a delivery force on the material prior to or simultaneous with contact of the device with the surface of an eye. As described herein, the force element is self-contained in the device or is integrated on the body of the device. As described herein, the material for administration is a fluid or semisolid material that flows under pressure. The material for administration may be pre-loaded in the device during manufacture. The material for administration may be placed into the reservoir portion of the device by the user, in a manner similar to a syringe, through a connector, valve or septum in fluid connection to the reservoir. The device may be configured to preload a force element such as a compression spring acting on the plunger or push rod providing a delivery force on the material for administration in the reservoir upon placement of the material for administration in the device. Other mechanisms may be provided for activating or priming the delivery force. For example, the delivery force may be activated by a mechanism to compress the force element from the exterior of the device. In another option, the delivery force may be activated by mechanically releasing a constrained force element or gas prior to use. A trigger, latch or other control means for the activation of the device can be located at various locations on the device.

The size of the reservoir, needle and plunger or push rod may be sized appropriately for the volume of material for administration to be delivered. For the solid or semisolid material for administration, the needle and potentially an extension of the needle into the body of the device may act as the reservoir. The needle, reservoir and plunger or push rod may be sized for solid or semisolid delivery volumes ranging from, for example, 0.1 microliters to 100 microliters.

The needle comprises a stiff material with a diameter to allow the material for administration to pass through the lumen of the needle, typically in the range of 20 gauge to 40 gauge (for example, less than 0.91 mm outer diameter / 0.6 mm inner diameter), where the length of the needle is suitable to both reach the intended tissue and provide sufficient volume for the desired dose of the composition to be administered. The needle is fixed to the body or barrel of the device and generally does not slide or move in relation to the body to provide precise control of needle depth during penetration of tissues. The needle may extend proximally into the body of the delivery device to provide sufficient volume for the material for administration. If additional volume is required for delivery of a semi-solid active agent containing composition, the proximal end of the needle may interface to a small reservoir within the body of the device that is pressurized by the delivery force element.

The distal tip of the needle may be beveled or sharpened to aid penetration. The bevel angle may be designed to facilitate entry into a specific target. For example, a short bevel of 18 degree bevel angle may be used to deliver into narrower spaces such as the subconjunctival or sub-Tenon's space. A medium bevel needle of 15 degree bevel angle may be used to deliver into spaces such as the suprachoroidal or supraciliary space. Longer bevels, such as 12 degree bevel angle may be used to deliver into the anterior or posterior chambers.

As described herein, the distal element is designed with a complementary bevel in a lumen of the distal element to provide close apposition of the distal seal to the needle bevel. The bevel of the needle is in alignment with the bevel in a lumen of the distal element. The most distal portion of the distal element may be flat or beveled to aid orientation of the needle during tissue penetration to aid reaching certain delivery targets. For example, a beveled or angled tissue contacting surface of the distal element may aid targeting of administration into the tissue targets with less depth such as the subconjunctival space, sub-Tenon's space and in some regions of the suprachoroidal space. The angle of the tissue contacting surface of the distal element may range from 90 degrees from the axis of the distal element for perpendicular insertion, to 45 degrees from the axis.

In some applications, it may be desired for the distal tip of the needle to direct the material for administration at an angle from the long axis of the needle. Such a design reduces force of the material for administration on the underlying tissues such as the ciliary body or choroid and may also be used to direct the material for administration in a desired direction such as toward the posterior region of the suprachoroidal space near the macular region of the retina. As described herein, the distal tip of the needle may be curved in the range of 5 to 60 degrees to direct the material for administration. As described herein, the distal tip of the needle may have an inner deflecting element in the lumen of the needle in the region of the bevel of the needle. The inner deflecting element may be a protrusion, a sloped surface or a ramp to direct the material for administration away from the long axis of the needle. The inner deflecting element may be located along the entire length of the needle bevel or in a discrete location. As described herein, the inner deflecting element is positioned at a specific distance from the proximal end of the bevel where the distance is 20% to 80% of the length of the needle bevel, 25% to 75% of the length of the needle bevel or 30% to 60% of the length of the needle bevel. The inner deflection element may have a width equal to the inner diameter of the needle, 75% to 50% of the inner diameter or 50% to 20% of the inner diameter. The inner deflection element may have a height of 75% to 50% of the inner diameter, 50% to 25% of the inner diameter or less than 25% of the inner diameter. The inner deflecting element may be formed by mechanical or thermal deformation of a region of the needle wall. The inner deflecting element may be formed by insertion and bonding of a shaped element within the lumen of the needle. As described herein, the inner deflecting element may be a protrusion that acts to fragment a solid material for administration at the needle distal tip to promote distribution of the material for administration in a tissue space such as the supraciliary space or suprachoroidal space.

The needle may be constructed from a metal, ceramic, high modulus polymer or glass. The length of the needle in tissue is selected to match the target location for the administration and the variation in target location due to anatomical variability. The effective full length of the needle is the length of the needle distal tip to the distal surface of the tissue interface, when the distal element has achieved full proximal travel. The distal element moves slidably on the needle during needle advancement, allowing for progressive increase in the length of needle protruding through the distal element during advancement into tissue. The material for administration is delivered automatically once the needle reaches the appropriate location which may be less than the effective full length of the needle. The release of force and resultant time for administration occurs quickly, in approximately 0.1 to 2 seconds depending on the properties of the material for administration and the amount of force from the plunger force element. The time for administration may also be controlled by a damping or frictional mechanism coupled to advancement of the plunger or push rod to limit the speed of the plunger. The release of force from the force element communicates to the physician with visible and tactile feedback that there is no need for additional advancement of the needle. The rapid delivery event gives the physician sufficient time to halt needle advancement, resulting in an effective variable needle length to accommodate patient to patient differences in tissue thickness. The variable needle length and self-actuation of administration is especially useful for administration into spaces that are not normally open spaces, such as the subconjunctival space, sub-Tenon's space, suprachoroidal space and supraciliary space. For the subconjunctival space and sub-Tenon's space the needle effective full length is in the range of 0.35 mm to 2 mm depending on the angle of needle insertion. For the suprachoroidal space and supraciliary space, the needle effective full length is in the range of 1 mm to 4 mm depending on the angle of insertion. For the vitreous cavity, the needle effective full length is in the range of 10 to 15 mm. The effective full needle length may, for example, be 0.3 mm to 3 mm, 0.35 to 2 mm, 1 mm to 4 mm, 10 to 15 mm.

As described herein, the distal element applies a distally directed sealing force against the tissue surface to maintain a seal on the surface of the eye. The sealing force is designed to be sufficient to seal the flow of the material for administration from the needle track during needle insertion into tissue. The sealing force is minimized to prevent compression of the tissues of a normally closed space or nearly closed space such as the suprachoroidal space or supraciliary space at the site of administration that would prevent delivery into the space or increasing the intraocular pressure that would restrict delivery of the material for administration into the space. As described herein, the distal element maintains contact with the tissue surface but does not apply a distally directed sealing force against the tissue surface to maintain a seal on the surface of the eye. As described herein, the distal element contacts the surface of the eye during penetration of the distal seal of the distal element by the distal tip of the needle but does not maintain contact with the surface of the eye after needle penetration through the distal seal and into ocular tissue. The tissue interface and distal seal may comprise a soft polymer, rubber or other material that allows needle penetration without coring of the material. The tissue interface and distal seal material is selected to provide compliance to both seal to the surface of the eye during insertion of the needle into ocular tissue and also to seal the delivery pathway from the needle until the needle is advanced through the distal seal. Once the needle penetrates the distal seal, the needle is advanced through the outer ocular tissues to reach the desired administration site. The tissue interface and distal seal remain on the surface of the eye. The distal seal is sufficiently resilient to prevent rupture by the material for administration under pressure prior to advancement of the needle through the distal seal. The portion of the distal seal in the path of the needle is also sufficiently thin to allow penetration by the needle without undue application of force. The distal seal is typically in the range of 250 to 1500 microns in thickness in the region that is penetrated by the needle.

As described herein a sealing force is provided by a compressible or collapsible element between the body of the device and the proximal end of the distal element or distal housing. As described herein, the tissue interface provides a sealing force by compression of the tissue interface or elastically compressible elements in the distal element. As described herein, the distal element is configured to allow an elastic reduction in length during needle advancement to apply a sealing force. As described herein, a friction element disposed in or about the distal element increases the force required to move the distal element proximally thereby promoting contact of the tissue interface with the surface of the eye and maintaining a seal against the eye surface during needle advancement. The friction of the distal element against the needle may be tailored in relation to the proximal movement of the distal element during needle advancement. An increase in friction may be obtained by increased contact or surface texture between the distal element and the external surface of the needle or through a decrease in the durometer of the distal element in order to tailor the amount of force applied by the tissue interface during proximal travel of the interface along the needle length. The friction may be varied along the path of travel of the distal element along the needle. High friction may be provided during the initial path of travel of the distal element to promote contact of the tissue interface to the surface of the eye during initial insertion of the needle into ocular tissues, the friction may be reduced after a length of the needle corresponding to the length of the needle bevel is inserted into ocular tissue. The length of travel of the distal element under the influence of the region of high friction is in the range of 0.3 mm to 2 mm.

As described herein, the distal element is attached to the body of the device by one or more collapsible elements. The collapsible element is configured to not allow an increase in length to prevent the distal seal from being displaced from the tip of the needle due to the delivery force applied to the material for administration prior to penetration of the distal seal. The collapsible element allows a reduction in length, thereby allowing proximal travel of the distal element during advancement of the needle into tissues. As described herein, the collapsible element comprises one or more elongated struts that may deform, bend or fold away from the needle during proximal travel of the distal element. As described herein, the collapsible element comprises a section of tubing concentric to the needle that has been cut to form openings along the axial length of the tubing to form collapsible struts. The shape and configuration of the collapsible struts may be tailored to provide a desired force-displacement characteristic of the collapsible element. The force versus displacement may be linear or non-linear. As described herein the material for administration is a gas, fluid or semisolid and the collapsible element provides a sealing force which transitions from an increasing spring like force per unit displacement to a constant force independent of displacement to keep the tissue interface and distal seal in sealing contact to the eye surface without undue application of force with further needle advancement into the eye.

Application of force above 80 to 100 grams-force may limit the ability of the material for administration to enter a closed space such as the suprachoroidal or supraciliary space. As described herein, the tissue interface applies a sealing force in the range of 40 to 80 gram force. The transition to a constant force is designed to occur after a length of the needle bevel is inserted into ocular tissue, corresponding to a compression or collapse of the collapsible element of 0.3 mm to 2 mm. As described herein the material for administration is a solid and the collapsible element provides for contact of the tissue interface to the surface of the eye during initial insertion of the needle into ocular tissue, but collapses to provide little or no resistance to proximal movement of the distal element along the needle after the bevel of the needle is fully inserted into tissue. The collapsible element may be assembled from components in a tubelike configuration or alternatively cut from a segment of tubing such as a laser machined nickel titanium alloy (e.g. Nitinol) tube. The collapsible element may be disposed between the elongate body and the distal element, such as between the barrel and the housing of the distal element (if present). The collapsible element may be fixed to the body of the device and to the distal element such that the distal element is proximally slidable on the needle but will not travel distally from its initial position to maintain the position of the distal seal while under delivery pressure.

As described herein, the tissue interface provides a sealing function. The sealing force provided by the tissue interface is within a range to provide sealing of the needle tract, but less than the force that would close the tissue space to impede movement of the material for administration into the space. A tissue interface with a tissue contacting surface area in the range of 0.45 to 5.07 mm² is suitable for sealing of the needle tract. Suitable materials for the tissue interface and distal seal include, but are not limited to, natural rubbers, silicone rubbers and thermoplastic elastomers such as polyurethanes. The stiffness of the rubber or elastomer may be selected to provide the appropriate combination of conformance to the tissue surface and sealing of the lumen of the distal end of the needle. The selection of the material of the tissue interface may also minimize the sealing force that might impede movement of the material for administration into the tissue space. The rubber or elastomer must also be capable of penetration by the distal tip of the needle to trigger release of the material for administration. Rubbers or elastomers with a Shore A durometer of 10 to 70, 10 to 50 or 10 to 30 are suitable for use as the sealing element. Suitable materials for a distal housing include, but are not limited to, polypropylene, polyethylene, polycarbonate, polysulfone, polyetheretherketone, acrylonitrile butadiene styrene, polystyrene, polyamide, and polyurethanes. Suitable materials for a distal collapsible element include, but are not limited to, stainless steel, spring temper steel, super-elastic nickel titanium alloys, cobalt chrome alloys, oil-tempered chrome silicon, polyimide, and polyetherimide. As described herein, the barrel of the device contains the reservoir and provides an external surface for holding the device during use. The reservoir may comprise a tubular cylinder attached on the distal end to the proximal end of the needle, with a plunger slidably disposed in the lumen of the tubular body. The reservoir may also provide for insertion of a cartridge containing the material for administration where the plunger or push rod of the device moves a slidable seal in the proximal end of the cartridge to deliver the material for administration. The body may be fabricated from a variety of thermoplastic materials suitable for medical use such as polypropylene, polyamide, polycarbonate, polysulfone, polyethylene, cyclic polyolefins, polystyrene and polymethylmethacryate. The body may incorporate external features such as textures or finger indentations to allow a user to more ergonomically grip and use the device. The body may incorporate index or measurement markings to provide an indication of the amount of material being delivered or to indicate that administration is complete. The body may incorporate transparent materials or a section of transparent material to allow the visualization of the material for administration in the reservoir or movement of the plunger or push rod to visually indicate the administration event. The plunger or push rod may have markings to aid visualization of reservoir loading and release of material for administration. The body may incorporate a label or indicator to provide the user with the orientation to the direction of the delivery, for instance a notation of the orientation of the needle bevel or the direction in which a deflection element will deflect the material for administration.

As described herein, the device comprises a means for providing a delivery force. The means as described herein could be, for example, a syringe with a compressible reservoir that can be "squeezed" or compressed by a user (directly or indirectly) to effect delivery of the material for administration. Alternatively, the means is a plunger or push rod with a biasing means or force element (such as a compression spring or a pressurized gas).

The device may be disposable and/or for single use. Alternatively, the device may be reusable.

As described herein, the device provides for the delivery of a controlled amount of gas as a first material for administration to facilitate the subsequent administration of an active agent containing composition to the suprachoroidal space or supraciliary space. A small amount of gas has been found to aid opening of a normally closed space near the needle tip, while the compressible nature of the gas minimizes potential trauma and pain. The delivery device comprises a gas reservoir and a flow path between the reservoir and the needle lumen, where the gas is introduced to the needle lumen proximal to the distal seal. The gas is pressurized with a force element such as a spring, compressed bladder, or expanding element. The gas reservoir is separate from the reservoir for the delivery of the active agent containing composition to prevent mixing of the gas with the active agent composition and to provide independent delivery characteristics. The gas reservoir may use the same force element as used for the delivery of the active agent containing composition or may have a separate, independent force element. The gas may comprise air, nitrogen, carbon dioxide, argon or a fluorocarbon. The gas may be selected for its solubility in the physiological environment to tailor the residence time of the gas. Generally, a fast dissolving gas such as air, nitrogen or carbon dioxide is used to minimize physical perturbation to the eye. The gas reservoir and gas flow path is configured to deliver gas volumes of 10 to 200 microliters, 20 to 150 microliters, 30 to 100 microliters, or 40 to 75 microliters of gas at standard temperature and pressure. The gas may be pressurized in a range of 0.1 to 2.0 bar, 0.15 to 1.6 bar or 0.2 to 1.0 bar gauge pressure. The volume and pressure of the gas, and the flow resistance of the gas flow path may be adjusted to tailor the timing of gas delivery from 0.1 to 2 seconds, 0.2 to 1.5 seconds, 0.3 to 1 seconds, 0.5 to 0.75 seconds.

The gas reservoir is activated prior to or simultaneous with penetration of the distal seal by the needle and advancement of the needle tip into tissues. The gas reservoir is activated by compression of the volume of gas in the gas reservoir by the action of a force element onto a plunger acting on the gas volume. The force element may be a spring or an expansive gas. The flow path for the gas may comprise the needle lumen, a portion of the needle lumen or a separate lumen. As described herein, the flow path comprises a hollow member external to the needle and the needle has at least one hole along the shaft of the needle that is in communication with the external hollow member to allow the gas to flow from through the hollow member and into the needle lumen. As described herein, the flow path comprises an outer sleeve that is coaxial to the needle and the needle has at least one hole along the shaft of the needle within the coaxial outer sleeve. The gas flows from the gas reservoir through the outer sleeve and into the needle lumen through the hole or multiple holes and out from the distal tip of the needle. Holes in the needle are located approximately 4 to 10 mm from the tip of the needle. Each hole may have a variety of shapes, including circular, polygonal or rectangular. The effective cross-sectional area of each hole may be from .05 to 2.0 mm². The coaxial outer sleeve may comprise a metal such as stainless steel or a plastic such as polypropylene, polyethylene, polycarbonate, polysulfone, polyethylene teraphthalate, polyetheretherketone, acrylonitrile butadiene styrene, polystyrene, polyamide, and polyurethanes. The coaxial outer sleeve is dimensioned to provide a gap between the inner surface of the sleeve and the outer surface of the needle for gas flow, where the gap is in the range of 0.1 to 2.5 mm. The length of the coaxial outer sleeve is configured to be compatible with the effective needle length of the device and may be in the range of 2 to 20 mm. The hole or holes may be made in the needle by laser machining, grinding, or punching.

The resultant gas flow path through the coaxial outer sleeve and into a needle hole bypasses any active agent containing composition in the proximal end of the needle to provide an unrestricted gas flow path to the distal end of the needle. A distal seal, tissue interface and distal element as described for the delivery of an active agent containing composition may be configured on the delivery device to facilitate the self-actuated delivery of the gas prior to the delivery of the active agent containing composition. Subsequent to the delivery of the gas, the active agent containing composition is administered through the needle to the suprachoroidal space or supraciliary space. As described herein, the plunger or push rod for the active agent containing material is incorporated into the plunger for the gas compression. As described herein, the plunger or push rod for the active agent containing material is separate from the plunger for the gas compression. The delivery of the active agent containing composition may be triggered manually after gas delivery or the device may incorporate a mechanism to administer the active agent containing composition after all or a portion of the gas has been delivered through the needle. The mechanism may comprise means to block or inactivate the delivery mechanism for the active agent containing composition by the gas pressure in the device in a primed or activated state, and release of the gas pressure by the delivery of gas releases the delivery mechanism for the active agent containing composition. The mechanism may comprise a mechanical linkage from the force element mechanism for gas compression to the force element mechanism for delivery of the active agent containing composition, a linkage from the gas compression plunger to the force element mechanism for delivery of the active agent containing composition or a linkage from the gas compression plunger to the plunger or push rod for delivery of the active agent containing composition.

The distal seal acts to prevent escape of the material for administration whether gas or active agent containing composition, from the needle or reservoir when the device is primed by activation of the delivery force before the distal seal is penetrated by the needle. This can be achieved by a seal between the needle lumen and the outside of the device. This seal may be achieved by the seal being in direct contact with the needle tip or may be achieved by using a distal element housing that is suitably sized to provide a liquid and gas tight seal around the needle shaft when placed over the needle tip. For example, the outer diameter of the needle may be complimentary to the inner diameter of the housing to provide a seal. As described herein, for delivery of a solid material, the seal may only block enough of the needle lumen so as to prevent the material from being expelled until the seal is moved proximally, thereby exposing the opening of the lumen.

The person skilled in the art will appreciate the difference between flowable, semisolid and solid materials for administration. Any material for administration may be described as flowable if, for example, the kinematic viscosity of the material is less than about 0.002 m² / sec at 20 °C. A material for administration may be described as semisolid if, for example, the kinematic viscosity of the material is greater than about 0.002 m² / sec at 20 °C.

Generally speaking, and as described above, the device is primed or activated when a pressure or force is placed on the material for administration, whether gas or an active agent containing composition, such that once the distal seal is penetrated by the needle and the needle reaches the desired site of delivery in the eye (such as the suprachoroidal space or supraciliary space), the material for administration is automatically released. The device is primed prior to or simultaneous with penetration of the distal seal. In this way, the device can be operated with one hand. The only force that needs to be applied by the user is the penetration force to allow the needle to penetrate the distal seal and then the eye tissue. The needle length can be suitably designed to target specific administration sites at corresponding depths in the eye. As described herein, the device may comprise a retaining means to retain the distal element on the needle once the device is primed.

Prior to delivery of the active agent containing composition or gas prior to the active agent containing composition, the distal element will generally not be in direct physical contact with the elongate body or the barrel. In fact, the distance between the proximal end of the distal element and distal end of the elongate body or barrel (and design of any compressible or collapsible element that may be present) can be arranged to determine the maximum depth of needle penetration. For example, during operation of the device, as the distal seal is pressed against the eye, the distal element and elongate body or barrel will move towards each other. It is this motion that advances the needle tip towards and through the distal seal/tissue interface and into the patient's eye. Once the proximal end of the distal element abuts against the distal end of the elongate body or barrel (or once the compressible or collapsible element does not permit further motion), continued advancement of the needle is prevented. Hence, the distance between the proximal end of the distal element and distal end of the elongate body or barrel may be equal to the maximum depth of needle penetration. Account may need to be taken for any distance between the needle tip and the distal seal/tissue interface and/or the use of any compressible element. In particular, the maximum depth of needle penetration may be determined by the distance between the proximal end of the distal element and distal end of the elongate body or barrel less the distance between the needle tip and the distal seal/tissue interface. Thus, the position and sizes of the distal element, needle, and distance between the needle tip and distal seal/tissue interface (if any) can be configured to determine a maximum needle penetration depth. The skilled person could design the device accordingly based on the present disclosure.

In this way the device may comprise a means for determining a maximum needle penetration depth to control the depth of penetration of the needle into the eye. The means can be a set distance between the proximal end of the distal element and distal end of the elongate body or barrel (as determined by the relative size of the distal element, the needle, the distance of the needle tip from the distal seal/tissue interface, and the shape and configuration of any compressible or collapsible element present). Alternatively, the needle may comprise a separate element that halts advancement of the distal element along the needle during operation (such as an element present on the needle disposed between the distal element and the elongate body or barrel, for example an annular ridge or clamp). As described herein, this element to prevent further advancement of the distal element along the needle during operation may be moveable such that the maximum needle penetration depth can be determined by the user. As described herein, the needle may comprise markings to allow the use to select an appropriate maximum needle penetration depth. As described herein, the maximum depth of needle penetration may be determined by the compressible element, for example the compressible element only allowing the desired needle penetration depth by way of increasing rigidity as the element is compressed, or by other mechanical means, such as entrapment of the compressible element between the proximal end of the distal element and distal end of the elongate body or barrel. The present disclosure therefore provides devices having fixed maximum needle penetration depths suitable for targeting the tissue space of interest. Suitable designs to achieve a fixed maximum needle penetration depth would be apparent to the skilled person based on this disclosure. Of course, the maximum depth of needle penetration can be within certain tolerances. Maximum needle penetration depth is also referred to herein as effective needle length.

As described herein, the active agent containing composition is preloaded in the delivery device, whereby the delivery device serves as the storage container for the composition prior to use. As described herein, the preloaded device is sterilized for use after placement and sealing of the active agent containing composition in the delivery device. The sterilization may be accomplished by established methods of sterilization such as heat or ionizing radiation. As described herein the active agent containing composition is preloaded in the device as a dry material that is reconstituted with a liquid that is introduced into the device prior to use. The delivery device may contain a port or connector in fluid communication with the device reservoir to facilitate reconstitution of the active agent containing composition within the delivery device.

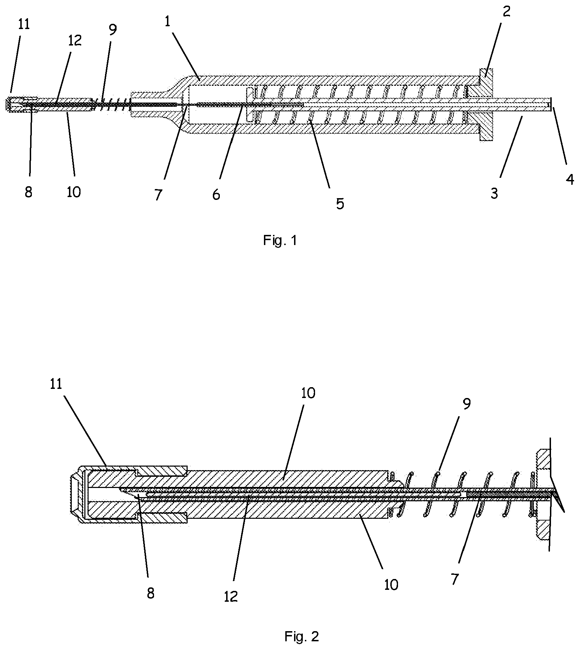

As described herein, the device is configured to deliver a material for administration, where the material for administration is an elongated solid material, semisolid material or implant within the lumen of the needle. Referring to the device depicted in Fig. 1 and the distal tip detail of the device in Fig. 2, the device comprises a hollow barrel 1, with a proximal barrel end cap 2. A plunger 3 slidably passes through the end cap. The plunger has a proximal end 4 which is sealed. A push rod guide tube 6 is slidably disposed in a lumen in the plunger 3, which provides support for a push rod 7 to prevent the push rod 7 from buckling during delivery. A plunger compression spring 5, provides a distally directed force on the plunger 3 and push rod 7. A beveled needle 8 is attached and fixed to the distal end of the barrel 1 such that the needle 8 does not move in relation to the barrel 1 to provide direct control of the location of the needle 8 tip when manipulating the position of the barrel 1. The distal end of the push rod 7 resides within the lumen of the needle 8 and moves distally when the tissue interface and distal seal 11 is opened by the distal tip of the needle 8. The distal element for the needle 8 comprises a tubular distal housing 10 surrounding the distal end of the needle 8. The tissue interface and distal seal 11 is attached to the distal end of the distal housing 10. A collapsible element is comprised of a distal housing spring 9 and is placed between the distal end of the barrel and the proximal end of the distal housing 10 to provide a distally directed force on the distal housing 10 thereby pressing the tissue interface and distal seal 11 onto the tissue surface.

As the needle 8 is advanced to penetrate the tissue interface and distal seal 11, the distal housing 10 moves proximally toward the barrel 1 while the needle 8 is advanced into tissue. The distal housing spring 9 acts to maintain pressure of the tissue interface and distal seal 11 as the needle 8 is advanced into tissue. While the tip of the needle 8 is passing through the outer tissues of the eye, the material for administration 12 within the lumen of the needle 8 is under pressure from compression spring 5 and the path from the distal tip of the needle 8 has been opened, but there is no tissue space for the material for administration 12 to be delivered from the needle tip. Once the distal tip of the needle reaches the desired space such as the suprachoroidal space or the supraciliary space, the material for administration 12 can exit from the needle and is expelled into the space. Figs. 1 and 2 show the distal segment of the device in an uncompressed state. The tissue interface and distal seal 11 and the distal housing 10 are disposed at the end of the uncompressed distal spring 9. The distal spring 9 is anchored to the barrel 1. As the device is deployed, the distal segment of the device transitions to a compressed state. The force of advancing the device into the tissue causes the distal spring 9 to compress, allowing the distal housing 11 and distal seal and interface 11 to slide proximally along the needle 8. The distal tip of the needle 8 penetrates the tissue interface and distal seal 11.

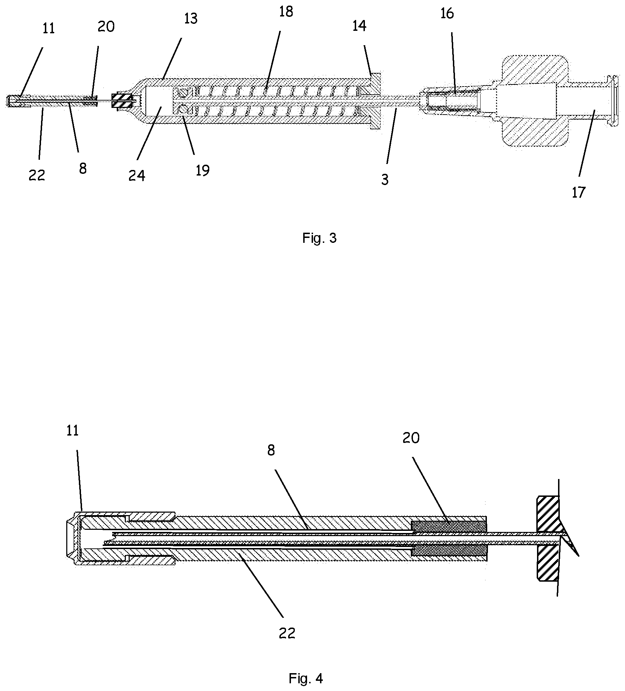

As described herein, the device is configured to deliver a material for administration where the material for administration is a fluid or flowable semisolid material. Referring to the device depicted in Fig. 3 and the distal tip detail of the device in Fig. 4, the device comprises a hollow barrel 13, with a proximal barrel end cap 14. A plunger 3 slidably passes through the end cap and has a plunger seal 19 within the barrel 13. The plunger seal 19 prevents leakage of material for administration between the plunger 3 and the barrel 13 inner wall. The distal end of the plunger 3 forms the proximal end of the reservoir 24. The shaft of plunger 3 has a flow path from the distal end, through the plunger seal 19 into the reservoir 24. The proximal end of the flow path is connected to a female Luer fitting 17. A one-way check valve 16 is placed between the female Luer fitting 17 and the proximal end of the flow path in shaft of plunger 3. A beveled needle 8 is attached and fixed to the distal end of barrel 13 such that the needle 8 does not move in relation to the barrel 13 to provide direct control of the location of the needle 8 tip when manipulating the position of the barrel 13. The plunger 3 moves distally when the tissue interface and distal seal 11 is opened by the distal tip of the needle 8. The distal element of the device comprises a tubular housing 22 surrounding the distal end of the needle 8. The tissue interface and distal seal 11 is attached to the distal end of the distal housing 22. A frictional element in the shape of a tube 20 is disposed in the proximal end of the housing 22 to contact the exterior of the needle 8 and provide means for the tissue interface and distal seal 11 to provide sealing at the administration site. To use the device, the fluid or flowable material to be delivered is placed into the device through the female Luer fitting 17 into reservoir 24. As the fluid or flowable semisolid material is placed in the reservoir 24, the plunger travels proximally, compressing the compression spring 18. The plunger compression spring 18, provides a distally directed force on the fluid or flowable semisolid material, pressurizing the reservoir 24. The check valve 16 provides a proximal seal to the flow path in the shaft of plunger 3 preventing flow out of the female Luer fitting 17. As the needle 8 is advanced to penetrate the tissue interface and distal seal 11, the distal housing 22 moves proximally toward the barrel 13. The tissue interface and distal seal 11 is placed on the surface of an eye and the device is advanced toward the eye. The tissue interface and distal seal 11 is compressed on the surface of the eye and the distal end of the needle 8 is advanced through the distal seal. While the tip of the needle 8 is passing through the outer tissues of the eye, the pressurized material for administration in the reservoir 24 has no tissue space to exit the needle tip. Once the distal tip of the needle reaches the target which allows for material flow into a tissue space, such as the suprachoroidal space or supraciliary space, the material can exit from the tip of the needle 8 and is expelled from the reservoir 24 into the space or cavity.

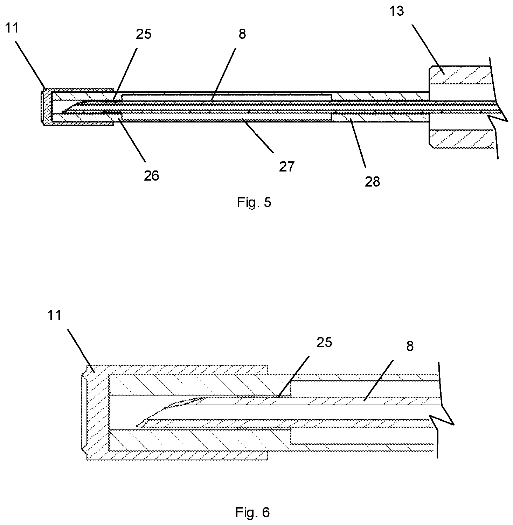

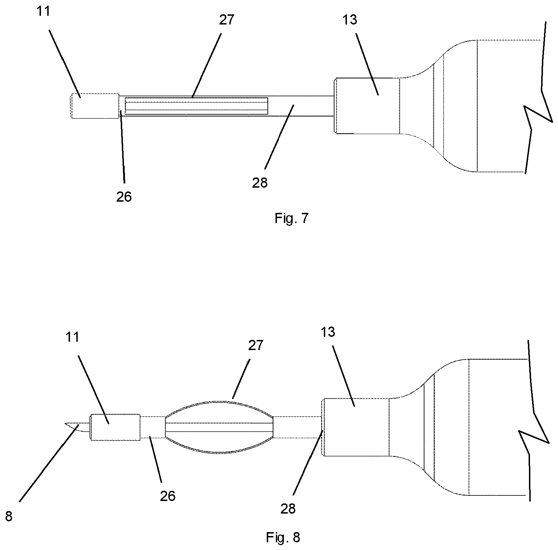

As described herein, the distal tip of the device is comprised of collapsible elements. Referring to the device depicted in Fig. 5 and the magnified device distal tip detail in Fig. 6, the distal tip is comprised of distal segment, a central collapsible segment and a proximal segment. The tissue interface and distal seal 11 is disposed about a distal tubular shaft 26. The inner lumen of the distal tubular shaft 26 contains an internal seal 25 which seals the space between the tubular distal shaft 25 and the beveled needle 8. The central segment is comprised one or more segments 27 which function as collapsible elements that can impart a force against the tissue surface during use. The collapsible elements 27 are attached or integral to the distal tubular shaft 26 and proximal tubular shaft 28. The proximal tubular shaft 28 is connected to the barrel 13 of the device providing an anchor point for the collapsible element and preventing distal movement of the tissue interface and distal seal 11. Fig. 7 shows the distal segment of the device in an uncollapsed state. The tissue interface and distal seal 11 and the distal tubular shaft 26 are disposed at the end of the collapsible elements 27. The proximal tubular shaft 28 is anchored to the barrel 13. Fig. 8 shows the distal segment of the device in a collapsed state. The force of advancing the device into the tissue causes the collapsible elements 27 to deform, allowing the distal tubular shaft 26 and tissue interface and distal seal 11 to slide proximally along the needle 8. The distal tip of the needle 8 has penetrated the tissue interface and distal seal 11.

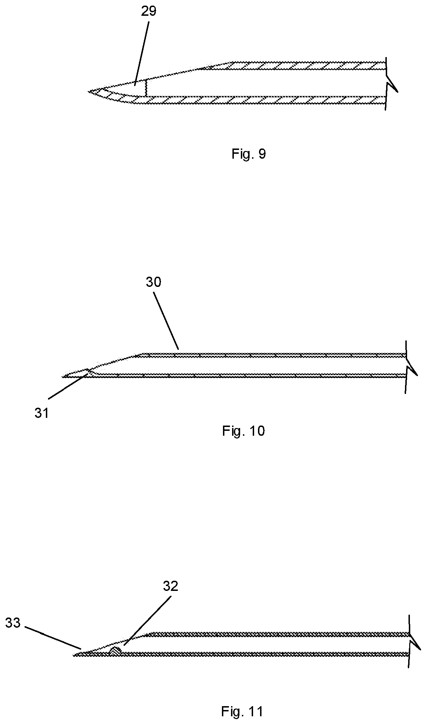

As described herein, the distal tip of the needle is configured to direct the material for administration at an angle from the long axis of the needle. Referring to the needle tip depicted in Fig. 9, the distal tip of the needle 29 may be curved to direct the material for administration. Referring to the needle tip depicted in Fig. 10, the distal tip of the needle 30 may have an inner deflecting element 31 in the lumen of the needle in the region of the bevel of the needle. Referring to the needle tip depicted in Fig. 11, inner deflecting element may be a protrusion 32 that acts to fragment a solid material for administration at the needle distal tip 33 to promote distribution of the material for administration in a tissue space, such as the supraciliary or suprachoroidal space.

As described herein, the delivery device is designed for delivery of a controlled amount of gas to facilitate the administration of a material comprising an active agent to the suprachoroidal space or supraciliary space. The delivery device comprises a gas reservoir and a flow path between the reservoir and the needle lumen, where the gas is introduced to the needle lumen proximal to the distal seal.

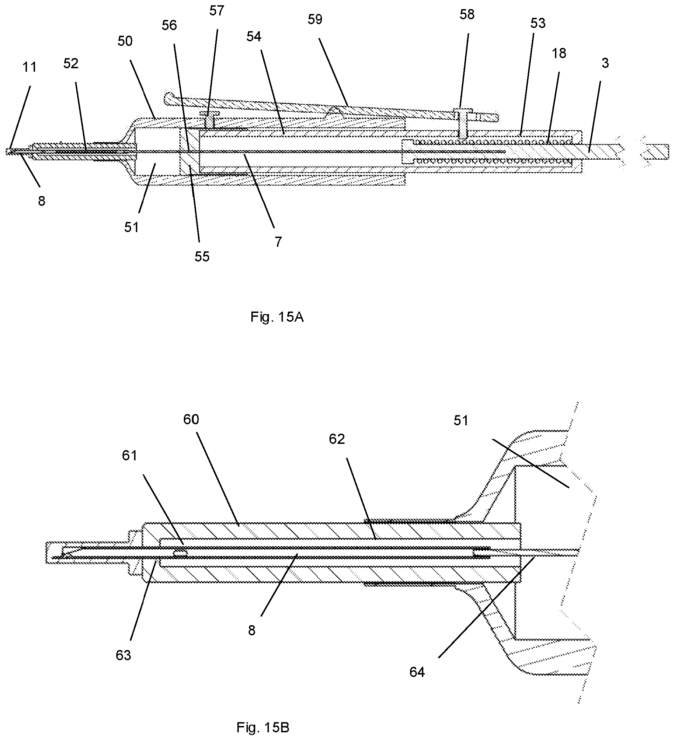

As described herein of the device depicted in Figs. 15A and 15B, a needle assembly, a gas reservoir with a plunger element and a force element with a push rod element which are coaxially configured. Fig. 15A depicts a schematic of the device with a distal segment consisting of a needle 8 with a distal seal 11, attached to the body 50 of the device and in communication with the gas reservoir 51. A solid material for administration 52 resides within the lumen of the needle 8. The body 50 contains a gas reservoir mechanism and a force element mechanism 53. The gas reservoir mechanism comprises a tubular barrel section 54 within which resides a sliding gas plunger 55. The plunger 55 is sealed about the inner surface of the tubular barrel 54 and contains a through hole 56 on the central axis which is sized to allow a gas tight seal on a push rod 7. When the plunger 55 is moved distally, the sealed plunger acts to compress any gas within the lumen of the barrel 54 thereby pressurizing the device for delivery of the gas. A distal locking pin 57 serves to hold the plunger 55 in place to maintain pressurization during preparation and use of the device.

The force element mechanism 53 is located in the tubular barrel section 54 and sized to allow free passage within the gas reservoir 51 in the barrel 54. The distal end of the force element 53 is attached to the proximal end of the gas reservoir plunger 55. The force element 53 is mechanically coupled to the push rod 7 attached to a sliding plunger 3 at the proximal end. The push rod 7 traverses through the gas reservoir plunger 55 and extends distally to align with the lumen of the needle 8. A compression spring 18 is placed over the sliding plunger 3 to provide the motive force for the push rod 7 to expel the material for administration 52. A proximal locking pin 58 is attached to a finger-actuated lever 59 and serves to hold the force element spring 18 in a compressed state during preparation of the device for use.

Fig. 15B depicts the distal end of a device according to Fig. 15A further comprising an outer sleeve 60 or jacket which allows for gas passage along the outside of the needle shaft 8. The needle 8 contains one or more holes 61 in the outer wall of the needle which allow the gas to enter the needle lumen near the distal tip of the needle 8. A gap 62 exists between the inner surface of the sleeve and the outer surface of the needle 8. The gap is sufficient to allow for unimpeded gas flow to the needle holes. The sleeve 60 is sealed against the needle 8 at the distal end 63 and is in open communication with the gas reservoir 51 at the proximal end 64.

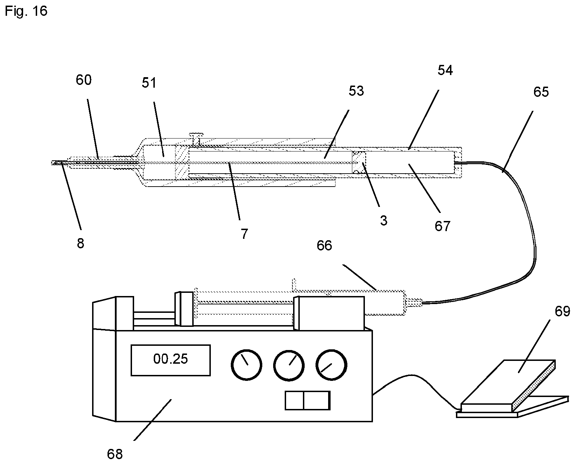

As described herein depicted in Fig. 16 a device consists of a distal needle 8 and outer sleeve 60, a gas reservoir 51 and a force element mechanism 53. The needle, outer sleeve and gas reservoir are as depicted in Figs. 15A and 15B. The mechanism for the force element to deliver a material for administration comprises a push rod 7 attached to a plunger 3 within a tubular barrel 54. The plunger 3 is slidably sealed against the inner surface of the barrel 54. The proximal end of the barrel 54 is configured to allow the attachment of tubing 65 which is connected to a plunger driven syringe reservoir 66. The syringe reservoir 66, tubing 65 and proximal end of the force element barrel 53 are filled with a non-compressible fluid such as water or oil 67. The gas in the gas reservoir 51 is compressed and delivered to through the lumen of the needle 8. Subsequently, the plunger of the syringe reservoir 66 is activated, the hydraulic mechanism serves to advance the force element plunger 3 and therefore the push rod 7 to expel the material for administration from the distal tip of the needle 8. The syringe reservoir 66 may be mounted on a syringe pump 68 that is manually activated by means of a button or foot switch 69 in order to trigger delivery of the material for administration. The rate of hydraulic advancement of the force element plunger 3 and push rod 7 may be adjusted to provide the rate of delivery of the material for administration from the distal tip of the needle 8.

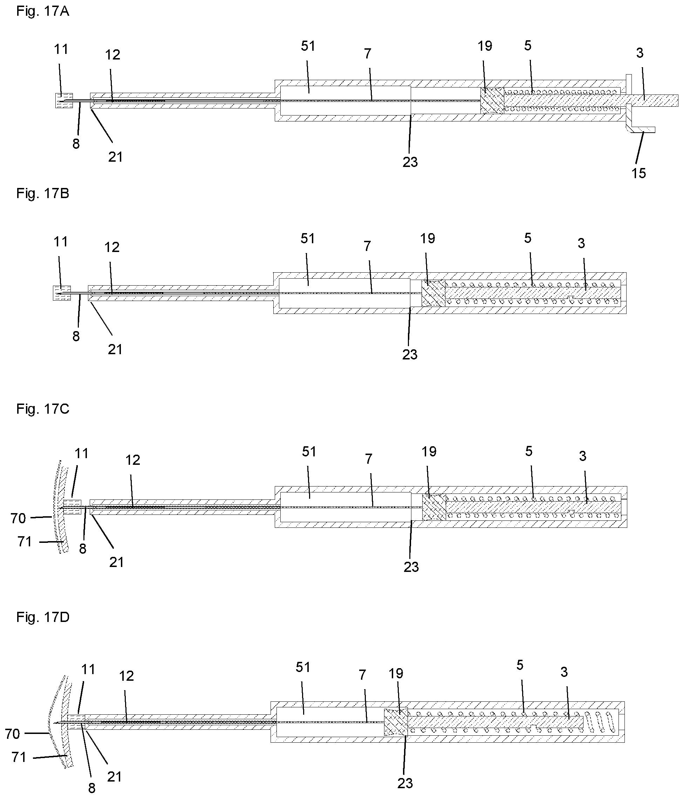

As described herein of the device depicted in Figs. 17A to 17F, the reservoir for gas delivery 51 is coaxial to the push rod 7 that applies a force on active agent containing composition 12 within the needle 8. The gas delivery portion of the device comprises a plunger seal 19 that moves to compress a volume of gas in the gas reservoir 51 between a distal seal 11 and the plunger seal 19. Fig. 17A depicts the device in a standby state prior to activation where the plunger rod 3 either does not yet have force applied to it or has a plunger stop feature 15 resisting the applied force to prevent the push rods axial movement towards the active agent containing composition 12. The user is able to remove a plunger stop feature 15 which allows the compression spring 5 to extend, displacing the plunger seal 19. The displacement of the plunger seal 19 compresses the enclosed gas volume in the gas reservoir 51 between the plunger seal 19 and the distal seal 11 until the plunger seal 19 reaches the equilibrium position as depicted in Fig. 17B. The plunger seal 19 stops at an equilibrium position once the compressed gas pressure exerts a force that equals the force applied by the compression spring 5 minus any friction (i.e. forces are balanced). The device is now activated to self-actuate the delivery of gas once the tip of the needle 8 has passed through the distal seal 11.

For use, the distal seal 11 of the device is placed at the administration site against the surface of the outer layers of an eye 70. By advancing the device toward the eye the needle 8 pierces through the distal seal 11 and at least partially through the outer layers of the eye 70 to reach the stage shown in Fig. 17C. For suprachoroidal or supraciliary administration the outer layers 70 would represent the conjunctiva and sclera, while the secondary layer 71 would represent the choroid or ciliary body. At the stage in Fig. 17C the gas in the enclosed volume remains at an elevated pressure and remains sealed by the outer layers 70 and/or the distal seal 11. By advancing the needle further into the eye, the needle 8 penetrates deeper through the outer layers 70 until the pressurized gas in the enclosed gas reservoir 51 is able to pass through the needle 8 and inflate the space between tissue layers 70 and 71. The expansion of this gas allows the plunger seal 19 to advance, as the pressure in gas reservoir 51 has now reduced allowing the spring 5 to extend. The device incorporates a needle stop face 21 to prevent the user from pushing the needle further through the outer layers 70. Fig. 17D shows the stage in which a portion of the gas has been delivered to separate the tissue layers 70 and 71. The plunger seal 19 has moved beyond a gas bypass feature 23 and is no longer sealing the enclosed gas reservoir 51 to allow a small volume of gas to be delivered before allowing a longer plunger and push rod translation for administration of the active agent containing composition to occur without continued gas delivery, this also eliminates the plunger seal friction for the remaining travel. Alternatively, maintaining a near constant cross-section (and therefore seal) throughout the travel of the plunger seal 19 without a bypass feature 23 enables larger volumes of gas to be delivered and a continuation of gas delivery during the active agent composition delivery.

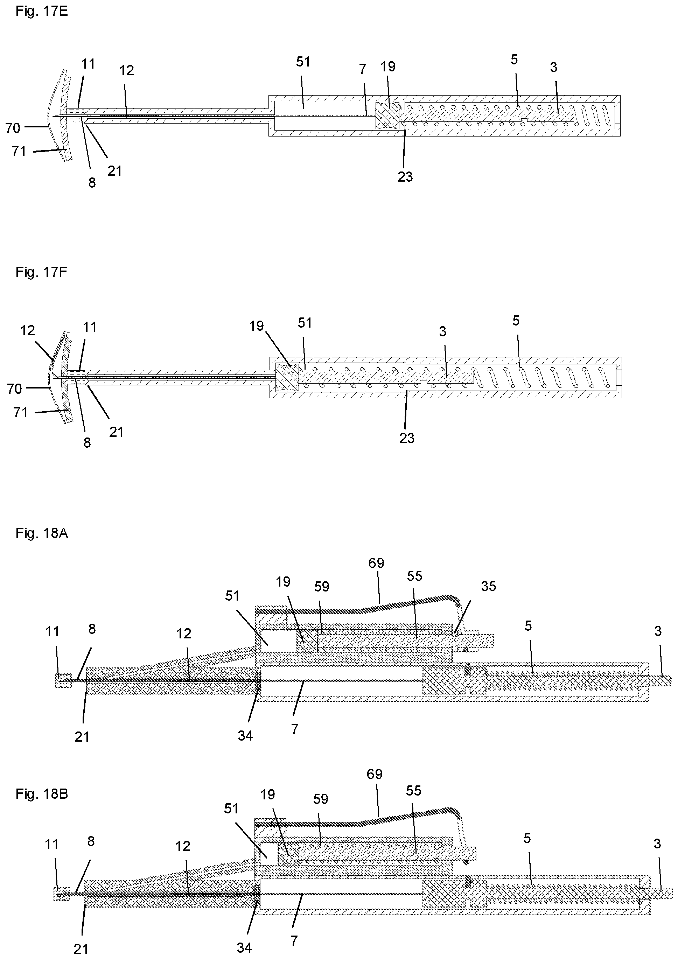

The time during or after gas delivery that the active agent containing composition 12 is delivered is able to be controlled by the length and positioning of the active agent containing solid or semi-solid elongated body 48 and push rod 7. Fig. 17E depicts the push rod 7 contacting the proximal end of the active agent containing composition 12. Fig. 17F shows the push rod 7 advanced further down the needle 8 bore to deliver the entirety of the active agent containing composition 12 to the space between the outer layers 70 and the secondary layer 71.

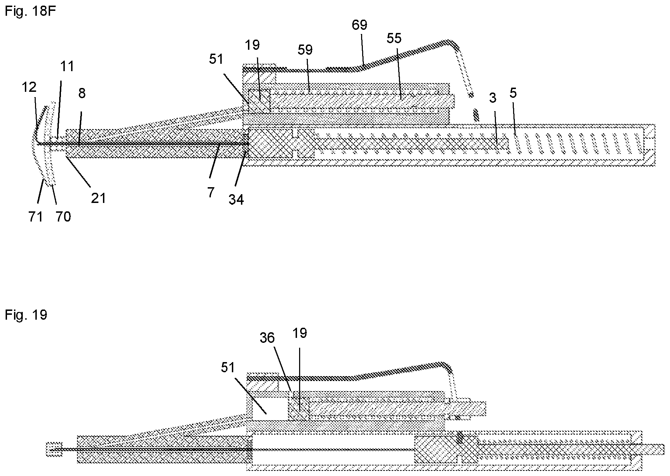

As described herein, the gas delivery portion of the device is configured to be independent of the push rod and delivery mechanism for the active agent containing composition. Fig. 18A depicts the device in standby configuration, where the plunger seal 19 for the gas delivery is uncoupled from the push rod 7 for delivery of the active agent containing composition. In this example, the gas compression spring 59 is being held in a compressed state by an activation latch 35. The drive spring 5 to deliver the active agent containing composition is being held in a compressed state by a mechanical couple, shown here as a bent metal wire 69 that has a position that is dependent on the travel of the plunger seal 19 and/or the gas plunger rod 55. The wire is biased away from the push rod 7 but in Fig. 18A it is shown to be initially held in this position by a loop around the gas plunger rod 55. The wire 69 could essentially be any feature that obstructs the elongation of the drive spring 5 until the plunger seal 19 has reached a displacement that allows the obstruction to be removed. Releasing the gas compression spring 59 by removing activation latch 35 compresses the enclosed volume to reach an equilibrium position shown in Fig. 18B where the force from the gas pressure acting on the plunger seal 19 plus friction is equal to the force from the gas compression spring 59. The pressurized gas reservoir 51 volume is enclosed by the distal seal 11, the plunger seal 19 and a push rod seal 34 which forms a seal between the push rod 7 and the proximal end of the needle lumen.

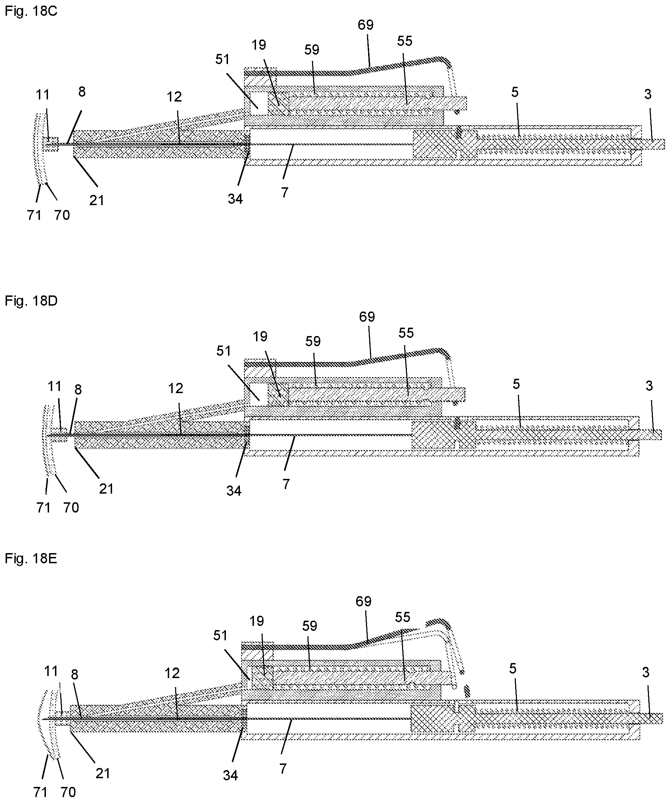

With the device in its equilibrium state the distal seal 11 is placed against the administration site on the surface of an eye. Advancing the device into the eye causes the needle 8 to pierce through the distal seal 11 and the outer layer of the eye 70. In the position shown in Fig. 18C the outer layers of the eye 70 and distal seal 11 are maintaining a seal on the enclosed gas reservoir 51 volume. Advancing the needle 8 further through the distal seal 11 and the outer layers 70 allows the pressurized gas in enclosed gas reservoir 51 to expand, travel down the needle 8 and inflate the space between layers 70 and 71 as is shown in Fig. 18D. The expansion of the gas reduces the gas pressure in gas reservoir 51 which allows the gas spring 59 to elongate displacing the plunger seal 19 and delivering a further volume of gas. The gas plunger rod 55 is attached to the plunger seal 19 and as this moves it reaches an unlatch point where the biased feature of the mechanical couple 69 is able to deflect out of the way so that it no longer obstructs the movement of the plunger 3 attached to the push rod 7 to deliver the active agent containing composition. This mechanism could be achieved in a number of ways but the critical aspect is that the displacement of plunger seal 19 removes an obstruction that is preventing the drive spring 5 elongation. Fig. 18E shows that the plunger seal 19 has reached the end of its travel and finished delivering gas. The push rod 7 has pushed the active agent containing composition 12 out of the needle 8 and into the space between layers 70 and 71.

In the device depicted in Fig. 19, the gas reservoir comprises a vent hole as a form of gas plunger seal bypass. A vent hole 36 is open from gas reservoir 51 to atmosphere, allowing the device internals to remain at atmospheric pressure between manufacture and use which may alleviate issues such as pressure changes during transit. The vent hole 36 can also be used to set the starting position for when the gas in reservoir 51 begins to be compressed. This enables the plunger seal 19 to move from any position in the standby state and the volume of gas that will be compressed and later delivered to the administration site is not compressed until the plunger seal 19 passes vent hole 36 and completes the seal on gas reservoir 51. The plunger seal 19 must pass the vent hole 36 between the standby state and the equilibrium position. Optionally the vent hole 36 can incorporate a filter to maintain sterility within the gas reservoir 51.

The described devices may be used in combination to deliver a fluid, semisolid or solid. The configuration of the distal portion of the device comprises the distal element which functions as a tissue interface and distal seal on the distal end of the needle. The use of a distal compression spring, frictional element, a collapsible element, or a combination of such elements in conjunction with the distal element may be used for delivery of a material for administration where the material is a gas, fluid, semisolid, solid or implant.

For use in the device, a lubricant may be used to aid delivery. The lubricant may be used to coat the material for administration or the needle lumen. The lubricant may also be placed in the lumen of the distal element to coat the tip of the material for administration and the outer surface of the needle as it passes into tissue. Suitable lubricants include, but are not limited to, oils, waxes, lipids, fatty acids and low molecular weight polymers. Low molecular weight polymers include, but are not limited to, polyethylene glycol and polysiloxane.

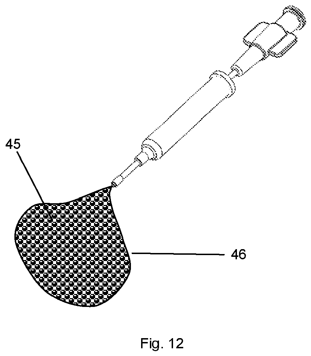

The material for administration may be a fluid, solid or semisolid composition of an active agent for delivery into the suprachoroidal space, supraciliary space or other spaces of the eye such as the vitreous cavity, subconjunctival space, sub-Tenon's space and sub-retinal space. The active agent may be solubilized in the fluid, semisolid or solid composition, or alternatively distributed in the composition as particles. As described herein, the composition comprises a plurality of drug-containing particles 45 formed into a semisolid composition 46 as a material for administration, shown schematically in Fig. 12. For delivery of the semisolid composition in the suprachoroidal space or supraciliary space, the composition is placed into the eye from the outer surface of the eye through a needle to preferentially locate the material in the suprachoroidal space or supraciliary space near the administration site. After placement in the suprachoroidal space or the supraciliary space, the semisolid composition transforms, degrades or dissolves into individual drug-containing particles that may migrate in the space. The semisolid mass of drug particles allows a large amount of drug to be administered in a very small volume to prevent an acute increase of intraocular pressure such as occurs with administration of an equivalent amount of drug suspended in a fluid. The semisolid formulation enables an effective amount of drug to be delivered in the range of 5 to 100 microliters, 10 to 75 microliters or 20 to 50 microliters.

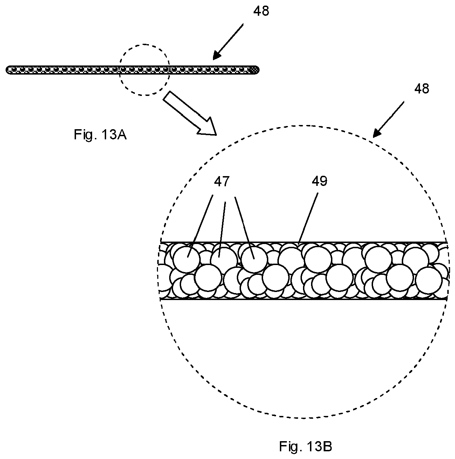

As described herein, the material for administration comprises a plurality of drug containing particles 47 formed into a formed elongated solid or semi-solid body 48, shown schematically in Figure 13A and Figure 13B. The formed elongated solid or semi-solid body 48 comprising the plurality of drug containing particles 47 may be in the shape of an elongated body such as a plug, tube or cylinder. The elongated body may be molded, machined, printed or extruded from the material for administration. As described herein, the formed elongated solid is an elongated body with a diameter approximately the inside diameter of the needle used for placement of the formed solid in the tissue space. The diameter may range from 0.60 mm, corresponding to a 20 gauge needle, to 0.159 mm, corresponding to a 30 gauge needle. Depending on the dose of drug and drug content of the particles, the formed solid may have a length ranging from 1 mm to 50 mm (for example 1 mm to 25 mm). After placement in the suprachoroidal space or the supraciliary space, the formed solid composition transforms, degrades or dissolves into individual drug containing particles that may migrate in the space. The formed solid mass of drug containing particles allows a large amount of drug to be administered in a very small volume to prevent an acute increase of intraocular pressure such as occurs with administration of an equivalent amount of drug suspended in a fluid. The volume of the delivered formed elongated solid may range from 0.1 microliters to 10 microliters (for example 0.1 to 5 microliters).

The drug containing particles may be in the form of a selected size range of crystals of the drug. The drug containing particles may be in the form of microspheres by fabrication of the drug into the form of spherical particles or by the formulation of the drug with an excipient such as a polymer and fabricating microspheres from the combination. Microspheres containing drug may be fabricated by any of the known means for microsphere fabrication such as by spray drying, coacervation or emulsion processes. The use of a non-toxic polymer to hold drug within microspheres allows tailoring of the drug release rate by the polymer composition, drug content and size of the microspheres. Microspheres with a drug content of 10-90 weight % drug may provide appropriate drug release. The use of polymers of selected solubility allows both water soluble and water insoluble drugs to be incorporated into microspheres. Suitable polymers include, but are not limited to, non-toxic water soluble polymers such as polyvinylpyrrolidone, polyvinylpyrrolidone co-vinyl acetate, polyvinyl alcohol, polyethylene glycol and polyethylene oxide, biodegradable polymers such as polyhydroxybutyrate, polydioxanone, polyorthoester, polycaprolactone, polycaprolactone copolymers, poly lactic acid, poly glycolic acid, poly lactic-glycolic acid copolymers and poly lactic-glycolic acid - ethylene oxide copolymers, and biological polymers such as gelatin, collagen, glycosoaminoglycans, cellulose, chemically modified cellulose, dextran, alginate, chitin and chemically modified chitin.

Alternatively, drug containing particles of approximately spherical shape or other uniform shapes may be prepared by milling of larger drug particles or by controlled crystallization. Drug particles and drug-containing microspheres may also be individually coated with a polymer layer to form drug particles with an external surface coating or barrier coating. The coatings may comprise non-toxic water soluble polymers including, but not limited to, polyvinylpyrrolidone, polyvinylpyrrolidone co-vinyl acetate, polyvinyl alcohol, polyethylene glycol and polyethylene oxide, biodegradable polymers such as polyhydroxybutyrate, polydioxanone, polyorthoester, polylactic acid, polyglycolic acid, poly lactic-glycolic acid copolymers, acid terminated polylactic-glycolic acid copolymers, polylactic-glycolic acid-ethylene oxide copolymers, polylactic acid-polyethylene glycol copolymers, polycaprolactone, polycaprolactone copolymers and polycaprolactone-polyethylene glycol copolymers, and biological materials such as gelatin, collagen, glycosoaminoglycans, cellulose, chemically modified cellulose, dextran, alginate, chitin, chemically modified chitin, lipids, fatty acids and sterols.

As described herein, the plurality of drug containing particles is formed into a solid or semisolid with an excipient. Suitable excipients include, but are not limited to, non-toxic water soluble polymers such as polyvinylpyrrolidone, polyvinylpyrrolidone co-vinyl acetate, polyvinyl alcohol, polyethylene glycol and polyethylene oxide, biodegradable polymers such as polyhydroxybutyrate, polydioxanone, polyorthoester, polycaprolactone, polycaprolactone copolymers, polylactic acid, polyglycolic acid, polylactic-glycolic acid copolymers and polylactic-glycolic acid-ethylene oxide copolymers, and biological materials such as gelatin, collagen, glycosoaminoglycans, cellulose, chemically modified cellulose, dextran, alginate, chitin and chemically modified chitin. The solid or semisolid composition may be formulated with a mixture of different excipients. The drug containing particles are mixed with the excipient in a suitable solvent that dissolves or forms a dispersion of the excipient, but does not extract the drug from the particles or dissolve the particles. As described herein, a semisolid composition is delivered as a mixture, dispersion or suspension with a solvent. As described herein, the solid or semisolid composition is formed in a mold or extruded and allowed to dry to form a solid of desired dimensions for delivery. Ideal for delivery of the formed solid or semisolid composition is an elongated shape with an outer diameter sized to fit within the lumen of a small gauge needle, 20 gauge or smaller, corresponding to 0.60 mm diameter or smaller. As described herein, the formed solid or semisolid composition has an outer diameter sized to fit within the lumen of a 25 gauge or smaller needle, corresponding to a 0.26 mm diameter or smaller.

As described herein, the semisolid composition is dried, such as by lyophilization, critical point drying or air drying for rehydration prior to delivery. The semisolid composition may have excipients to aid reconstitution such as salts, sugars, water soluble polymers and surfactants.

As described herein, the drug containing particles are sized smaller than the inner diameter of the delivery needle to allow close packing of the drug containing particles within a formed solid or semisolid to enhance mechanical properties. Such drug containing particles would have an average diameter in the range of 4 to 100 microns, for example 4 to 25 microns, and may comprise a mixture of diameters to facilitate close packing. The mean or median diameter of the particles may be in the range of 2 to 100 microns, for example 2 to 50 microns, 2 to 40 microns, 2 to 30 microns or 2 to 20 microns.

The dispersion and migration of the drug containing particles are desired to promote a uniform distribution of the particles in the eye. The dissolution of the excipient and resultant release of drug containing particles may be triggered by the absorption of fluid from the tissue space, for example due to the ionic environment or the temperature of the environment. As described herein, the excipient comprises a lipid or fatty acid with a melting temperature between room temperature and the temperature of the ocular tissues space, approximately 37 degrees centigrade (for example, a melting temperature between 21 and 37 degrees centigrade, between 25 and 37 degrees centigrade, or between 30 to 35 degrees centigrade). The rate of release of the individual drug containing particles from the solid or semisolid composition may be tailored by the addition of hydrophilic or amphiphilic agents that increase the dissolution rate of the excipients of the solid or semisolid composition. The release of the drug containing particles may occur over hours, days or weeks, depending on the amount and composition of the material for administration. For example, a maximum (or minimum, depending on the formulation) of 50% of the drug containing particles may be released after 1 hour, 6 hours, 12 hours, 1 day, 3 days or 1 week.

The solid or semisolid composition may act by the ionic environment of the tissue space to provide dissolution, which may be provided by ionically crosslinked polymers such as sodium alginate. The solid or semisolid composition may be triggered for dissolution in the tissue space by temperature, such as with lipids and fatty acids with a melt transition temperature greater than room temperature, approximately 20 degrees centigrade, and less than or equal to the temperature within the ocular tissue space, approximately 37 degrees centigrade. Such lipids and fatty acids include, but are not limited to, capric acid, erucic acid, 1,2-dinervonoyl-sn-glycero-3-phosphocholine, 1,2-dimyristoyl-sn-glycero-3-phosphocholine, and 1,2-dipentadecanoyl-sn-glycero-3-phosphocholine and mixtures thereof.

Due to the small size of the drug containing particles, drug release from the particles may be too rapid to provide sustained drug effect after administration to the eye. It is an object of the disclosure to provide drug containing particles with prolonged release kinetics (i.e. controlled release formulations). As described herein the drug is incorporated into a polymer matrix that creates a poor diffusion path for the drug thereby slowing drug release as compared to the drug without a polymer matrix. As described herein, the drug containing particle is coated with a barrier such as a polymer or other compound. The barrier material typically has different chemical properties than the drug so that the drug is not readily soluble through the barrier coating and is slowed in drug release as compared to the drug particle without a barrier coating. One method for selection of the barrier coating is a material with a different partition coefficient or log P than the drug, with an increased difference providing an increased barrier to drug release. As described herein, the individual particles of a drug, are coated with a barrier coating of increased water solubility or decreased log P compared to the drug to form a barrier coating on each particle. Barrier materials may include, but are not limited to, acid terminated poly lactic-glycolic acid copolymers, polylactic acid-polyethylene glycol copolymers polycaprolactone-polyethylene glycol copolymers, polyethylene oxide, polyvinylalcohol, hydroxyethylmethacrylate, alginate, gelatin, chitin and glycosoaminoglycans. As described herein, the individual particles of a drug are coated with a barrier coating of decreased water solubility or increased log P compared to the drug to form a barrier coating on each particle including, but not limited to, a hydrophobic polymer, lipid, fatty acid or sterol. Drug particles may be coated by any of the known means for particle coating, for example, spray drying, electrostatic spraying or chemical deposition. As described herein, shown schematically in Fig. 13A and Fig. 13B, the formed solid or semisolid material 48 comprises a plurality of drug containing particles 47 encapsulated or coated with a barrier material 49, such as a soluble polymer or other coating, to modify the drug release characteristics and/or the mechanical properties.

While the drug of the composition is primarily contained in the plurality of particles, some drug may also be formulated into the excipient. The drug in the excipient may act to prevent extraction or diffusion of drug from the particles during processing or storage. The drug in the excipient may also act to provide a rapid release component to the drug formulation to initiate therapeutic effect of the drug while allowing the drug in the particles to provide a sustained delivery to maintain the treatment effect.

As described herein, the active agent containing composition comprises a drug and an excipient comprising a biodegradable or bioerodible material. The biodegradable or bioerodible material may be comprised of, for example but not limited to, polyhydroxybutyrate, polydioxanone, polyorthoester, polycaprolactone, polycaprolactone copolymer, polycaprolactone-polyethylene glycol copolymer, polylactic acid, polyglycolic acid, polylactic-glycolic acid copolymer, acid terminated polylactic-glycolic acid copolymer, or polylactic-glycolic acid-ethylene oxide copolymer, gelatin, collagen, glycosoaminoglycan, cellulose, chemically modified cellulose, dextran, alginate, chitin, chemically modified chitin, lipid, fatty acid or sterol. The drug may be dispersed in the biodegradable or bioerodible material as an amorphous solid dispersion. The drug may be dispersed in the biodegradable or bioerodible material as a plurality of drug crystals. The drug may be dispersed in the biodegradable or bioerodible material as both an amorphous solid dispersion and as drug crystals. The active agent containing composition is shaped as an elongate solid body for delivery into the ocular tissue space. Release of the drug from the elongated body allows the drug to diffuse into the tissues of the eye and may be assisted by the flow of fluid in the tissue space. In the case where the drug is in the form of a solid amorphous dispersion, the biodegradable or bioerodible material is selected to provide the desired drug loading and release characteristics of the drug. In the case where the drug is in the form of dispersed drug crystals, the amount of drug, the biodegradable or bioerodible material characteristics and the crystal form of the drug may be selected to provide the desired drug loading and release characteristics of the drug. The drug crystals may also be coated with an excipient to reduce the drug release rate of the active agent containing composition. As described herein, the composition has an extended release of the active agent or drug. The drug elution from the composition may have a half-life in the range of 14 to 180 days, 21 to 90 days or 30 to 45 days.

A variety of active agents and drugs may be delivered by the present disclosure to the eye for the treatment of ocular diseases and conditions including inflammation, infection, macular degeneration, retinal degeneration, neovascularization, proliferative vitreoretinopathy, glaucoma and edema. Useful drugs include, but are not limited to, steroids, non-steroidal anti-inflammatory agents, antibiotics, VEGF inhibitors, PDGF inhibitors, anti-TNF alpha agents, mTOR inhibitors, cell therapies, neuroprotective agents, anti-hypertensive agents, antihistamines, aminosterols and nucleic acid based therapeutics. The active agents and drugs may be in the form of soluble solutions, suspensions, gels, semisolids, microspheres, formed solids or implants.

As described herein, the material for administration is preloaded in the device during the time of manufacture prior to use. The source of force to provide a delivery force to the material for administration may be activated just prior to use. As described herein the activation is achieved by a mechanism to preload the force element, such as compressing a spring, from the exterior of the device such as by a movable proximal handle attached to the plunger. As described herein, the source of force is preloaded during manufacture when the drug is placed in the device and the preloaded force is stabilized by means of a stop mechanism. Prior to use, the stop mechanism is released, thereby placing the force on the material for administration prior to or simultaneous with contact of the eye and the delivery is triggered by the advancement of the needle through the tissue interface and distal seal. As described herein, the material for administration comprises a volume of gas prior to delivery of an active agent containing composition. Activation of the delivery mechanism for the active agent containing composition is triggered by the delivery of gas from a gas reservoir in the device immediately prior to the delivery of the active agent containing composition.

As noted, a variety of active agents and drugs may be delivered to the eye for the treatment of a variety of ocular diseases and conditions including inflammation, infection, macular degeneration, retinal degeneration, neovascularization, proliferative vitreoretinopathy, glaucoma, and edema. Useful drugs include, but are not limited to, steroids such as corticosteroids including dexamethasone, fluocinolone, loteprednol, difluprednate, fluorometholone, prednisolone, medrysone, triamcinolone, betamethasone and rimexolone; non-steroidal anti-inflammatory agents such as salicylic-, indole acetic-, aryl acetic-, aryl propionic- and enolic acid derivatives including bromfenac, diclofenac, flurbiprofen, ketorolac tromethamine and nepafenac; antibiotics including azithromycin, bacitracin, besifloxacin, ciprofloxacin, erythromycin, gatifloxacin, gentamicin, levofloxacin, moxifloxacin, ofloxacin, sulfacetamide and tobramycin; VEGF inhibitors such as tyrosine kinase inhibitors, antibodies to VEGF, antibody fragments to VEGF, VEGF binding fusion proteins; PDGF inhibitors, antibodies to PDGF, antibody fragments to PDGF, PDGF binding fusion proteins; anti-TNF alpha agents such as antibodies to TNF-alpha, antibody fragments to TNF-alpha and TNF binding fusion proteins including infliximab, etanercept, adalimumab, certolizumab and golimumab; mTOR inhibitors such as sirolimus, sirolimus analogues, Everolimus, Temsirolimus and mTOR kinase inhibitors; cell therapies such as mesenchymal cells or cells transfected to produce a therapeutic compound; neuroprotective agents such as antioxidants, calcineurin inhibitors, NOS inhibitors, sigma-1 modulators, AMPA antagonists, calcium channel blockers and histone-deacetylases inhibitors; antihypertensive agents such as prostaglandin analogs, beta blockers, alpha agonists, and carbonic anhydrase inhibitors; aminosterols such as squalamine; antihistamines such as H1-receptor antagonists and histamine H2-receptor antagonists; and nucleic acid based therapeutics such as gene vectors, plasmids and siRNA.

As described herein, there is provided the active agent containing composition of the disclosure for use in medicine, in particular for use in ocular medicine. As described herein, there is provided the active agent containing composition of the disclosure for use in the treatment of an ocular disease or condition. The ocular disease or condition may be inflammation, infection, macular degeneration, retinal degeneration, neovascularization, proliferative vitreoretinopathy, glaucoma or edema. The active agent containing composition is generally for administration by delivery through a delivery device of the present disclosure.

As described herein there is provided a method, not according to the invention and present for illustration purposes only, of treating an ocular disease or condition by administration of the active agent containing composition of the disclosure to the eye, for example to the suprachoroidal space or to the supraciliary space. The active agent containing composition may dissolve or transform into a plurality of spherical drug-containing particles that migrate from the site of administration (for example the suprachoroidal space or supraciliary space) after delivery. The active agent containing composition may be administered through a needle. As described herein, the active agent containing composition may be administered using the device described herein. The ocular disease or condition may be inflammation, infection, macular degeneration, retinal degeneration, neovascularization, proliferative vitreoretinopathy, glaucoma or edema.

One instance, not according to the invention and present for illustration purposes only, includes the use of an active agent in the treatment of an ocular disease or condition. Such uses therefore also include the use of an active agent in the manufacture of a medicament for treating an ocular disease or condition.

As described herein there is provided a method, not according to the invention and present for illustration purposes only, of treating an ocular disease or condition by administration of a controlled amount of gas prior immediately prior to administration of an active agent containing composition of the disclosure to the eye, for example to the suprachoroidal space or to the supraciliary space.

As described herein, there is provided a kit of parts comprising the delivery device described herein and the active agent containing composition of the disclosure. The active agent containing composition may be provided preloaded into the delivery device. Alternatively, the active agent containing composition may be provided as a plurality of discrete dosage forms suitable for insertion into the delivery device. Therefore a kit may also provide the active agent containing composition of the disclosure in the form of a plurality of discrete dosage forms along with the delivery device.

A number of examples are now described which are provided for illustrative purposes.

### Examples:

### Example 1: Solid Material Delivery Device with Inner Deflecting Element

A device was fabricated to administer a solid or semisolid material into the suprachoroidal or supraciliary space of the eye. A barrel element was fabricated by cutting off the proximal end of a 0.5 ml insulin syringe to a barrel length of 30 mm. The integral needle was removed from the barrel to allow the attachment of standard Luer hub needles. The distal tip of the barrel was cut off leaving a remaining section of Luer taper capable of securely holding a Luer hub needle. A barrel end cap was fabricated from a nylon 10-32 socket head cap screw with a thread length of 4.5 mm. A through hole of 1.86 mm diameter was drilled through the end cap to allow the plunger to freely slide through the end cap. A plunger shaft was fabricated from a tubular Teflon coated stainless steel rod with an outer diameter of 1.8 mm and an inner diameter of 0.8 mm and a length of 43 mm. The distal end of the shaft was turned down to a diameter of 1.74 mm and a stainless steel washer of 4.1 mm outer diameter, 1.70 mm inner diameter and 0.5 mm thickness was press-fit onto the rod to provide a distal stop for the plunger spring. The proximal end of the rod was drilled out to 1.55 mm diameter.

A polyetheretherketone (PEEK) monofilament 0.25 mm diameter and 80 mm long was used as a push rod element to expel the delivered material from the device. A segment of PEEK capillary tubing with an outer diameter of 1.57 mm, an inner diameter of 0.25 mm and a length of 2.25 mm was fabricated as a securement for the PEEK push rod . The lumen of the PEEK securement tube was sufficiently tight enough on the push rod to hold it securely in place under normal use, but allowed the push rod to be slidably adjusted. The push rod was inserted through the PEEK securement tube and then the assembly was press-fit into the drilled out proximal end of the plunger shaft with the push rod extending distally through the lumen of the plunger rod. A stainless steel hypodermic support tube with an inner diameter of 0.30 mm and an outer diameter of 0.61 mm was placed over the push rod in order to prevent kinking of the push element during deployment. The support tube was slidably disposed within the inner diameter of the plunger shaft. A compression spring with an outer diameter of 3.1 mm and a wire diameter of 0.18 mm and a length of 31.8 mm was placed over the shaft of the plunger and the barrel end cap was then slid over the plunger shaft proximal to the spring. The plunger and push rod assembly was placed into the barrel housing with the push rod extending through the distal tip of the barrel. The end cap was press fit into the barrel proximal end securing the plunger assembly within the barrel.

A safety mechanism was incorporated into the device to prevent premature activation of the plunger by the plunger spring force. Two shallow grooves 180 degrees apart and perpendicular to the axis of the plunger were made in the plunger at a distance of 19 mm from the distal tip. The distance between the groove faces was 1.5 mm. A securement clip was fabricated from brass sheet with a width of 6.3 mm and a length of 18 mm. A slot with a width of 1.6 mm and a length of 8.8 mm was machined into the securement clip. The slot was cut in the center of the short side of the securement clip and traversing in the long axis direction.

A needle was fabricated to allow a solid element to be delivered at an angle from the axis of the needle lumen. The needle was fabricated from 27 gauge ultra-thin walled hypodermic tubing with an inner diameter of 0.29 mm and an outer diameter of 0.41 mm. The needle was adhesively bonded into a standard female Luer needle hub. An inner deflecting element was fabricated in the needle by inserting a length of polyimide tubing with an inner diameter of 0.26 mm and an outer diameter of 0.28 mm. The segment of the polyimide tubing that extended out from the beveled tip of the needle was bent at a 90 degree angle to the axis of the needle lumen and then adhesively bonded in place. After the adhesive had cured, the polyimide tube was skived along the face of the needle bevel. The remaining curved tip of the polyimide tube allowed a solid material to exit the tubing at an angle of approximately 20 degrees from the axis of the needle.

A molded cylindrical tissue interface and distal seal element was fabricated from 70 Shore A durometer silicone rubber. The distal element had a length of 3.7 mm and a diameter of 1.75 mm. The distal element had a lumen of 2.7 mm length and 0.38 mm diameter. The distal end of the lumen of the distal element was configured with a beveled shape which conformed to the bevel on the distal end of a 27 gauge needle. The distal element was attached to the distal tip of the needle such that the needle bevel was in contact with the lumen bevel in order to seal the distal tip of the needle. The non-beveled section of the lumen acted as a slidable seal on the shaft of the needle and provided enough frictional force against the needle shaft to maintain the distal tip against the eye surface during advancement of the needle through the distal seal of 1 mm thickness.

For use, the plunger was retracted thereby compressing the plunger spring until the plunger grooves were exposed proximally to the end cap. The securement clip was placed over the plunger such that the slot on the securement clip engaged the grooves on the plunger shaft. The securement clip then was held against the proximal end surface of the end cap by the spring force, preventing movement of the plunger. The needle assembly was removed and a solid material was inserted into the proximal end of the polyimide insert tubing. The push rod was aligned in the insert tubing and advanced, pushing the solid material towards the distal end of the needle. The needle and barrel assembly were mated yielding a device ready for use.

### Example 2: Use of Solid Material Delivery Device with Inner Deflection Element

A device according to Example 1 was fabricated. A solid element for delivery was fabricated by extruding a slurry comprised of drug loaded microspheres in a carrier material. The drug loaded microspheres comprised polylactic-glycolic acid copolymer spherical particles in the range of 10 to 20 microns in diameter. The microspheres were loaded with 25 weight % fluocinolone acetonide, a corticosteroid. A slurry for extrusion was formulated using 85 weight % microspheres and 15 weight % binder. The binder was formulated from 92 weight % high molecular weight, K90 polyvinylpyrrolidone and 8 weight % low molecular weight, K12 polyvinylpyrrolidone, which was in a solution of 25 weight % concentration in de-ionized water. The slurry was dispensed using a 0.3 ml syringe with a distal needle of 0.25 mm inner diameter at a pump speed of 50 microliters/min using a syringe pump to extrude filaments of similar diameter to the inner diameter of the dispensing needle deflection element. The filaments were allowed to dry at ambient conditions prior to further processing. The microsphere-containing filament was cut to a length of 10 mm and loaded into the delivery device as described in Example 1.

A cadaver porcine eye was prepared by inflating the posterior chamber to a pressure of approximately 20 mm Hg. A target location 5 mm posterior of the limbus of the eye was chosen for administration. The securement clip was removed from the plunger shaft. The tissue interface and distal seal was placed against the scleral surface and the needle tip was then advanced through the distal seal and into the tissues with the needle bevel oriented towards the posterior of the eye. Once the needle lumen reached the suprachoroidal space, the segmented solid element was free to exit the needle and was expelled by the push rod under the plunger spring force. The delivery of the solid element was confirmed by manually excising a flap in the sclera to expose the suprachoroidal space. A sample of the fluid in the suprachoroidal space was taken and placed on a microscope slide. Examination of the slide under the microscope at 100X magnification revealed numerous microspheres that had been released from the solid element administered into the suprachoroidal space.

### Example 3:

A solid active agent containing composition was fabricated for delivery in the form of an elongated body or filament. Two binder materials were prepared, polyethylene oxide (PolyOx WSR-301) of 7 million Daltons average molecular weight dispersed in deionized water at a concentration of 2.5% and K90 polyvinylpyrrolidone of 360,000 Daltons average molecular weight dissolved in deionized water at a concentration of 40%. The binders were mixed in a ratio of 66% polyethylene oxide and 33% polyvinylpyrrolidone. Microspheres with average diameter of 15 microns were mixed into the binder formulation at a concentration of 93.7 wt %. The microsphere contained 50 wt % dexamethasone and 50 wt % polylactic-glycolic acid copolymer. The composition was loaded into a 0.3 ml syringe with a modified distal tip. The distal tip comprised a polyimide tube with a lumen inner diameter of 0.24 mm. The syringe was placed on a syringe pump and the composition was extruded as a filament. The filament was allowed to dry at ambient conditions then cut to desired length. A coating material was prepared by dissolving K12 polyvinylpyrrolidone of 40,000 Daltons average molecular weight in ethanol at a concentration of 25 wt %. The lengths of filament were dipped into the coating dispersion and slowly removed then allowed to dry. Two dip coating layers were applied to the filaments. Once dry, the filaments were then cut to a final length of 20 mm appropriate for loading into the delivery device.

A delivery device was fabricated comprising a distal needle assembly, a gas reservoir portion and a force element.

The distal needle assembly comprised a 27 gauge thin walled needle with a deflecting feature coined into the wall of the needle opposite of the lumen of the needle. The deflecting feature was located approximately 0.6 mm proximal of the tip of the needle and extended approximately ½ the diameter of the lumen or 0.1 mm. The deflecting feature was configured to both deploy the filament off axis from the needle axis and to fracture the filament as it traversed passed the feature. The needle had a 15° bevel with a standard three bevel hypodermic configuration. Two oval side holes 0.5 mm long and 0.18 mm wide were placed approximately 7 mm proximal from the needle tip. The holes were laser cut through the wall of the needle tubing and were 180° apart. An outer sleeve was fabricated from nylon tubing 1.1 mm inner diameter and 1.3 mm outer diameter. The outer sleeve was bonded to the needle, distal to the side holes such that approximately 3 mm of needle extended beyond the sleeve. The gap between the sleeve and the needle allowed for the free flow of gas from the reservoir to the needle side holes and then to the needle tip. This assembly was bonded within a female Luer hub modified to accept the jacket outer diameter. A distal seal and tissue interface consisting of a Shore 50A durometer solid silicone rod segment 3 mm long and 0.75 mm in diameter was placed over the tip of the needle. The seal was gas tight and prevented premature expelling of the gas in the gas reservoir when pressurized. A length of filament was inserted into the proximal end of the needle lumen.

The main body of the device comprised a 6 ml polycarbonate syringe body as a gas reservoir and a 1 ml polycarbonate syringe as a housing for the force element. The distal end of the syringe comprised a male Luer lock connection. The 6 ml syringe rubber plunger tip was removed from the plunger shaft and mounted on a machined plastic body configured to hold the plunger tip on the distal end and to receive the distal end of the 1 ml syringe on the proximal end. In this manner, the 1 ml syringe was mounted coaxially within the 6 ml gas reservoir syringe.

The 1 ml syringe was configured as the force element section of the device. A stainless steel wire 0.23 mm in diameter was mounted to the tip of a plunger rod as a push rod. The plunger rod consisted of a polytetrafluoroethylene coated stainless steel tube with an outer diameter of 1.8 mm. A flange was fixed to the distal end of the plunger rod as the distal spring perch. A compression spring was placed over the proximal end of the plunger rod, which acted as the motive force for the deployment of the filament. The spring was fabricated from stainless steel wire 0.24 mm in diameter with a spring diameter of 2.6 mm, a free length of 80 mm and a compressed length of 18 mm. A cap was attached to the proximal end of the 1 ml syringe to provide the proximal spring perch. A through hole in the cap allowed the plunger rod to extend proximally so that it could be pulled rearward to compress the spring in preparation for use.

An external finger controlled lever was attached to the syringe body. The lever comprised a distal section, a proximal section and a central pivot area. The proximal section of the lever acted as an activation mechanism for a release pin. The release pin held the force element plunger, with the spring compressed, in preparation for use. When the plunger rod was pulled rearward to compress the spring, the release pin freely fell into place thereby holding the plunger rod from moving. The distal section of the lever comprised a through hole through which a gas reservoir locking pin was placed and was freely slidable within the hole. The reservoir locking pin fell into place when the gas reservoir plunger was pushed fully distally thereby pressurizing the system. Pressing the distal end of the lever raised the release pin allowing the force element to activate and the device to deploy the filament.

The device was activated by pulling the gas reservoir plunger distally until 100 to 150 microliters of air was held in the gas reservoir. The end of the plunger rod was then pulled rearward compressing the spring then the release pin was inserted to hold the spring compressed. The distal needle assembly which was loaded with the filament was tightly assembled onto the distal end of the device body. The plunger assembly was then advanced distally compressing the air within the main body and the reservoir locking pin was pushed into place to maintain pressure.

A porcine eye was prepared as described in Example 2. The distal tip of the device was pressed against the surface of the eye and advanced. The needle tip penetrated the distal seal and remained sealed as it entered the scleral tissues. Once the tip of the needle lumen entered the suprachoroidal space the air was released from the pressurized gas reservoir into the space. The gas delivery was confirmed through the change in tone in the eye by a finger resting on the surface of the eye. As soon as the air delivery was confirmed the lever was depressed thereby pulling the release pin, activating the spring force element and deploying the filament into the space. The device was then removed from the site. A scleral flap was made over the administration site and the sclera peeled back exposing the suprachoroidal space. The filament was observed and was consistently fractured into segments of approximately 0.5 mm length.

### Example 4. Low Sealing Force Tissue Interface

Tissue interfaces were fabricated in a manner similar to those described in Example 1 and Example 3. Two different outer diameters of tissue interface were fabricated: 1.75 mm diameter and 2.50 mm diameter. Samples of each diameter tissue interface were fabricated using four different durometers of liquid silicone elastomer, Shore 10A, 30A, 50A and 70A.

A test method was prepared to determine the sealing force of the various samples of the tissue interface. A segment of PEEK tubing 8.3 mm long was placed over a 27 gauge × 13 mm thin walled hypodermic needle to serve as a stop so as not to allow the tissue seals to travel proximally during the test. A tissue seal being tested was then placed over the needle tip. The length of the PEEK tubing was sized so as to allow approximately one-half of the needle bevel section to protrude through the tissue interface distal surface. A test surface was used which consisted of a silicone elastomer pad with a durometer of Shore 50A and 3.2 mm thick. The needle was mounted to a tee-fitting which in turn was mounted on the shaft of a digital force gauge with a 250N capacity mounted on a motorized test stand. The side leg of the tee-fitting was attached to a length of tubing and then to a Luer fitting and a three-way valve. A 10 cc syringe filled with water was attached to the valve. The syringe was held vertically using a ring stand. Tests were conducted using two different constant pressures which were generated by applying fixed weights of 1030 and 1656 grams respectively to the finger flange of the syringe plunger. The inside of the syringe had a cross-sectional area of 1.64 × 10-4 m2 which corresponded to fluid pressures of 6.18 × 104 Pa and 9.93 × 104 Pa respectively.

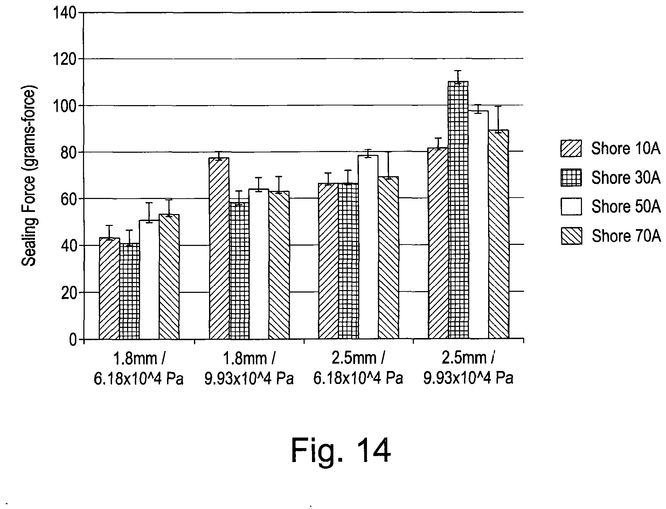

To perform a test, the needle tip was traversed down until the tissue interface was close to touching the silicone test pad. The test stand motor was jogged downward until approximately 30 grams-force of pressure was being applied to the tissue interface. The three-way valve was opened and the periphery of the tissue interface observed for water leakage. The valve was closed and then the needle was moved downward until approximately 35 grams-force of pressure was being applied. The valve was opened and the tissue interface observed for leakage. The tissue interface pressure on the test pad was increased in 5 gram-force increments in this manner until no leakage was observed, e.g. a seal was achieved and the force was recorded. The test was repeated with the second syringe pressure weight. The testing was performed on the two different tissue interface diameters and the four different durometers (Table 1 and Figure 14). Two samples of each tissue interface were tested three times each for a total of six data points for each test condition. The silicone test pad was moved after each test so that each needle penetration was at a new site. Figure 14 shows a tissue interface minimum sealing force graph grouped by diameter & fluid pressure, as a function of durometer. Table 1 shows the minimum Sealing Force in Grams-Force for Tissue Interface Test Samples (Average and Standard Deviation) grouped by Tissue Interface Diameter and Fluid Pressure, as a Function of Durometer.

**Table 1**

| **Durometer** | **1.8 mm Diameter 6.18 x 10⁴ Pa** | **1.8 mm Diameter 9.93 x 10⁴ Pa** | **2.5 mm Diameter 6.18 x 10⁴ Pa** | **2.5 mm Diameter 9.93 x 10⁴ Pa** |
|---|---|---|---|---|
| **10** | 43.3 ± 5.2 | 77.5 ± 2.7 | 66.7 ± 4.1 | 81.7 ± 4.1 |
| **30** | 40.8 ± 5.8 | 58.3 ± 5.2 | 66.7 ± 5.2 | 110.0 ± 4.5 |
| **50** | 50.8 ± 7.4 | 64.2 ± 4.9 | 78.3 ± 2.6 | 97.5 ± 2.7 |
| **70** | 53.3 ± 6.1 | 63.3 ± 6.1 | 69.2 ± 10.7 | 89.2 ± 10.2 |

### Example 5. Semisolid Active Agent Containing Composition

A semisolid active agent containing composition was prepared. A 1.5 wt % of polyethylene oxide (PolyOx WSR-303) of 7 million Daltons average molecular weight was dispersed in deionized water. Microspheres with average diameter of 15 microns were mixed into the polyethylene oxide dispersion at a concentration of 2.5 wt %. The microsphere contained 50 wt % dexamethasone and 50wt % polylactic-glycolic acid copolymer. The semisolid composition was stained with sodium fluorescein for observation.

A delivery device was fabricated to administer the semisolid composition into the suprachoroidal space of an eye. A body and attached needle was fabricated by cutting off the proximal end of a 1.0 ml insulin syringe with 12.7 mm long 27 gauge integral needle to a barrel length of 32 mm. The proximal open end of the syringe barrel was tapped for a 10-32 thread. A barrel end cap was fabricated from plastic with a through hole sized to fit the plunger shaft and an external thread of 10-32. A plunger was fabricated from a metal tube with an outer diameter of 2.4 mm and an inner diameter of 0.4 mm. The distal end of the plunger comprised two flanges welded to the end and with a gap of 1.3 mm between them. A silicone O-ring seal was placed between the flanges. A compression spring with a spring force of 0.98 N/mm, an outer diameter of 4.6 mm and a wire diameter of 0.4 mm was placed over the shaft of the plunger, and the barrel end cap was then slid over the plunger shaft proximal to the spring. The proximal end of the plunger comprised a larger diameter tube sized to allow the insertion of a rubber duck-bill style check valve which was welded to the plunger shaft after assembly of the plunger spring and end cap. The valve was inserted into the larger tube and a female Luer lock fitting was attached over the tube and valve.

A housing 9.5 mm long with a 1.5 mm outer diameter and a 0.35 mm inner diameter, was fabricated from polycarbonate tubing, with a sealing element disposed in the proximal end of the housing. The housing length was such that the distal tip of the housing extended 2 mm beyond the tip of the needle when assembled. A molded tissue interface and distal seal element 3.5 mm long with an outer diameter of 1.9 mm and an inner diameter of 0.9 mm was fabricated from 50 Shore A durometer silicone. The tissue interface and distal seal was placed over the distal end of the housing. A housing compression spring of 0.08 N/mm spring force, an outer diameter of 1.5 mm and a wire diameter of 0.1 mm was placed over the needle to provide sealing force against the tissues. The housing compression spring had a free length of 4.8 mm and a compressed length of 0.8 mm. The spring was placed over the needle, then the housing and tissue interface and distal seal were placed over the needle and the spring was adhesively bonded to the proximal end of the housing at one end and the syringe barrel at the other end.

One hundred microliters of the semisolid active agent containing composition was placed into the delivery device through the female Luer lock fitting, thereby placing a delivery pressure on the composition. An enucleated porcine eye was prepared by infusion to 20 mm Hg. The distal tip of the device was placed on the pars plana region of the eye and advanced into the eye. The delivery of material was observed to occur once the tip of the needle reached the appropriate depth to deliver the composition to the suprachoroidal space. After delivery, indirect ophthalmoscopy was performed, showing none of the composition in the vitreous cavity. Dissection of the sclera overlying the administration site revealed the semisolid composition in the suprachoroidal space, extending to approximately 4 mm from the administration site. A second porcine eye was prepared for administration. After administration, indirect ophthalmoscopy showed none of the composition in the vitreous cavity. The eye was perfused with phosphate buffered saline through a 30 gauge needle at a pressure of 20 mm Hg. After 18 hours, the perfusion was discontinued and the sclera at the posterior pole of the eye dissected to reveal the suprachoroidal space. A swab of the suprachoroidal space 9 mm posterior to the administration site was performed and rubbed against a glass slide for microscopic examination at 100X and 200X magnification. Microscopy demonstrated a plurality of microspheres retrieved from the posterior region of the suprachoroidal space.

### Example 6

A delivery device was fabricated. The device consisted of a distal assembly, a gas reservoir portion and force element to deliver the material for administration.

The distal assembly comprised a 27 gauge thin walled hypodermic needle with a 15 degree beveled tip. A deflecting feature was created in the wall of the needle by mechanically deforming the wall of the tube opposite the bevel using a circular punch. The deflecting feature was located 0.63 mm from the distal tip and had a height inside the needle lumen of 0.11 mm. Two oval side holes of 0.18 mm width and 0.5 mm length were cut by laser through the wall of the needle tubing. The holes were 180° apart and located 5.5 mm from the distal tip of the needle. An outer jacket for the needle was fabricated from PET tubing with an inner diameter of 0.94 mm and an outer diameter of 2.0 mm. The jacket element was bonded to the needle distally of the needle side holes. The gap between in the inner diameter of the jacket element allowed for the passage of gas from the gas reservoir into the needle lumen through the laser cut side holes. A distal seal and tissue interface was molded from silicone elastomer of 50A durometer. The seal was 3.0 mm long and had an outer diameter of 0.76 mm and an inner lumen of 0.30 mm diameter. The distal end of the seal was solid in order to provide a seal against the passage of the gas and the material for administration after activation of the device. The needle/jacket assembly was bonded into a female Luer lock fitting for attachment to the body of the device. An open passage existed through the Luer fitting and into the ID of the needle jacket to allow for gas flow from the gas reservoir, through the needle side holes and into the lumen of the needle.

The gas reservoir portion of the device was fabricated from a polypropylene 3 ml syringe barrel. The stock rubber syringe plunger tip was removed from the stock plunger and mounted on the distal end of a machined plunger with a length of 10 mm. The machined plunger contained a through hole of 0.38 mm diameter sized to slidably fit the plunger rod. The proximal end of the machined plunger was sized to fit the distal end of the force element portion of the device. A locking tab in the body of the 3 ml syringe was created by cutting a three sided rectangle in the plastic of the syringe. The locking tab was positioned such that when the plunger of the gas reservoir portion was advanced to the end of the barrel, the distal end of the tab could be depressed, thereby holding the plunger in place. In this manner, the plunger was used to pressurize the gas charge in the reservoir and the locking tab used to maintain the pressure for use. The distal end of the barrel incorporated a male Luer lock configuration for mounting of the distal assembly of the device.

A force element portion of the device was fabricated using a polypropylene 1 ml syringe barrel. The syringe barrel was mounted in a reverse configuration in the device, i.e. with the barrel proximal end directed to the device distal end. The proximal end of the barrel was modified in order to provide a friction fit to the machined plunger in the gas reservoir, thereby becoming the distal end of the force element portion. The distal end of the barrel retained the male Luer lock configuration for attachment to the force mechanism, thereby becoming the proximal end of the portion. The syringe plunger shaft was modified by cutting it to a length of 3 mm. A push rod of 60 mm length and 0.23 mm diameter was mounted to the plunger extending distally. The push rod was inserted through the gas reservoir machined plunger and plunger tip and extended sufficiently to engage the proximal end of the needle lumen. The plunger stopper provided a distal seal to the proximal segment of the syringe barrel which was used as a fluid reservoir for the force producing mechanism.

A hydraulic system was configured to produce the force for advancing the push rod in the force element portion of the device. A hydraulic system was used in order to allow for varying the speed of the push rod actuation. The hydraulic mechanism consisted of a syringe pump, syringe and tubing configured to attach to the Luer lock on the distal end of the force element. A 6 ml syringe fluid reservoir was mounted on a programmable syringe pump. A length of PTFE tubing with Luer fittings at each end was attached to the fluid reservoir and to a three-way Luer stopcock. The Luer stopcock was attached to the proximal Luer connection on the force element syringe. The fluid reservoir was filled with water. The stopcock position was adjusted to open the side port and the syringe pump was activated to fill the tubing and stopcock with water. The proximal lumen of the force element portion was manually filled with water such that there was no air bubble in the line between the fluid reservoir syringe and the force element plunger. In this manner the force element could be advanced by activation of the syringe pump. A foot pedal control was attached to the syringe pump to allow for manual activation of the deployment of the drug composition. The gas reservoir portion and the force element portion were assembled together, thereby creating a unified assembly as the main body of the device.

Prior to use, the force element portion was pulled rearward, sliding the gas reservoir plunger shaft proximally until a set volume of gas (air) was present in the gas reservoir. A volume of air in the range of 100 - 150 microliters was used, with an expected actual delivery of air being in the range of 75 - 100 microliters due to the dead volume of the system. A filament of a solid material for administration consisting of microparticles in a binder formulation was inserted into the proximal end of the needle lumen. The distal needle, jacket, Luer hub assembly with the material for administration was then attached to the main body, aligning the distal end of the push rod within the lumen of the needle. The force element portion was then pushed forward, moving the gas reservoir plunger until it stopped and the locking tab was pushed downward thereby maintaining the position of the gas reservoir plunger. In this manner, the air in the distal end of the device was pressurized with the distal seal on the needle tip preventing premature release of the air.

The distal tip of the device was pressed against the pars plana region of a cadaver porcine eye at an angle approximately 45 degrees from the normal vector from the surface of the eye. The device was advanced forcing the needle tip to penetrate through the distal seal and into the tissues of the eye. When the needle tip reached the suprachoroidal space, the pressurized air was released into the space thereby opening up the space to allow for the delivery of the drug composition. During placement and advancement of the device, the user held a finger against the eye to assist in maintaining stability. The air delivery was confirmed by the change in tone in the eye under the stabilizing finger. Immediately upon confirmation of the air delivery, the syringe foot pedal was activated which started the hydraulic system to advance the push rod and deliver the filament into the suprachoroidal space. After activation, the device was removed. A scleral flap was cut into the eye, revealing the suprachoroidal space and the microparticles of the drug composition was observed in the space.

### Example 7: Sequential automatic gas and solid material delivery device

A device was fabricated and tested for its ability to successfully deliver sequential gas and drug into the suprachoroidal space or supraciliary space of the eye. The device was configured so that the gas delivery portion of the device was independent of the push rod and delivery mechanism for the active agent containing composition.

A plastic distal chassis was manufactured by 3D printing from an acrylic polymer blend (Objet Inc., VeroWhite). The distal chassis was configured such that it held two 1ml Luer polycarbonate syringes parallel to each other, 12.5mm axis to axis apart and in a matching orientation.

The first syringe formed the gas reservoir and was fabricated by cutting off the proximal end of a 1 ml polycarbonate syringe and retaining the distal Luer end with an internal barrel length of 28.5 mm and an internal barrel diameter of 4.7 mm. A hole of 1.5 mm diameter was drilled radially into the side of the gas reservoir at a position such that once the stopper seal past the distal edge of the hole this sealed the syringe barrel from atmosphere and had a capacity of 110 microliters.

The inner surface of the proximal end of the gas reservoir was tapped with an M5 metric thread for an axial length of 5 mm. A plunger rod was turned from stainless steel; its diameter being substantially 1.6 mm. A modified elastomeric stopper (wherein one sealing face was removed to reduce the stopper friction), removed from the original plunger rod of the 1 ml syringe, was push fitted onto retaining features at the distal end of the turned stainless steel plunger rod. The stopper remaining distal circumferential seal had an axial length of approximately 0.5 mm. This plunger rod and stopper assembly was placed into the gas reservoir. A compression spring with a spring rate of 0.032 N/mm, free length of 31.75 mm and external diameter of 3.18 mm was positioned over the turned plunger rod. A 1.8 mm diameter hole was drilled axially along the entire axis of an M5 × 5.5 mm countersunk socket head screw; this screw was placed over the proximal end of the plunger rod, then screwed into the proximal end of the gas reservoir, thereby compressing the spring and biasing the plunger rod and stopper to the distal end of the syringe barrel. An activation latch was fabricated by cutting a "U" shaped section of 0.5 mm thick sheet metal with a central slot 1 mm wide. The stainless steel plunger rod had a notch of 0.9 mm diameter by 1 mm cut in 5 mm from the proximal end of the rod. The slot in the activation latch was fitted into the notch of the plunger rod to hold the compression spring at a compressed height of 10 mm with the activation latch butting into the countersunk screw at the proximal end of the cut syringe.

The second 1ml Luer syringe, called the drive syringe, was assembled into the distal chassis in coaxial configuration with the device needle. A slot was cut longitudinally into one side of this syringe; the slot being 3 mm wide and 15 mm long, running axially in a proximal direction from a position 27.5 mm proximal from the distal internal end of the syringe barrel. The slot in the drive syringe was oriented to face directly toward the gas reservoir.

A cantilever spring was fabricated by bending 0.8mm diameter spring steel wire. The form of this was such that the cantilever spring was affixed into a 0.8mm diameter blind hole in the distal chassis and was bent around the exposed proximal end of the stainless steel gas plunger rod and entered in through the slot in the drive syringe. In this position the cantilever spring was configured to exert a force of 1.4 N on the turned gas plunger rod and was biased away from the drive syringe.

A plastic push rod holder was fabricated with a diameter substantially 2.7 mm and a total length of 97 mm; the distal end of the push rod holder had a diameter of 4.45 mm for an axial length of 1.5 mm. The center of the distal end face of the push rod holder was drilled with a 0.5 mm diameter by 5 mm deep hole. A 0.2 mm diameter straight tungsten wire as a push rod was glued coaxially into this hole such that the wire extended 52 mm beyond the distal end face of the push rod holder. This push rod and push rod holder assembly was inserted into this drive syringe so that the push rod extended distally of the syringe Luer taper. A compression spring (the drive spring) was placed over the push rod holder; the spring had a rate of 0.02 N/mm, a free length of 180 mm and an outer diameter of 4.1 mm. The cantilever spring that extended radially inwards of the drive syringe inner wall obstructs the push rod assembly from moving distally.

The proximal chassis was fabricated as a plastic 3D printed part which was secured at the proximal end of the device. The proximal chassis had the functions of providing an end stop for the 180 mm long drive spring in the drive syringe and provided features for mounting a rotary damper. This proximal chassis was attached to a proximal end face of the 3D printed chassis by means of a single M5 by 12 mm cap head socket screw and an M5 nut; interlocking features prevented relative rotation of the 3D printed parts. By connecting the proximal and distal chassis the drive spring was compressed to a length of 44 mm. A rotary damper with a damping torque of 0.20 N-cm was attached via bolts to the proximal chassis; the damper comprised a fixed part and a 7.2 mm outer diameter ten toothed gear (module 0.6). An 80 mm long toothed module 0.6 plastic rack compatible with gear of the rotary damper engaged with the teeth of the rotary damper and was affixed to the proximal end of the push rod holder so that any axial translation in the push rod was resisted by the rotation of the rotary damper.

A needle assembly was fabricated with a 3D printed needle hub that housed a 27 gauge thin walled, 32 mm long needle with a 15° bevel. The needle was adhered in to the hub at the distal end with 3mm of needle extending from the hub; the adhesive formed a gas tight seal. The needle had two oval side holes of 0.18 mm width and 0.5 mm length were cut by laser through the wall of the needle tubing. The holes were 180° apart and located 5.5 mm from the distal tip of the needle. A length of 24 mm long, 0.020 in internal diameter and 1/16 in outer diameter microbore tubing was inserted over the needle to fill the void between the needle shaft and the 3D printed needle hub which also assisted in retaining the needle concentricity with the hub. The microbore tubing was positioned to not obscure the laser cut holes through the side of the needle. Extending through the wall of the needle hub was a 6 mm long length of 25 gauge stainless steel hypodermic tubing which was connected to a 0.19 mm internal diameter by 50 mm long length of flexible microbore tubing which at the opposing end was attached to a 25 gauge Luer needle to connect the gas syringe flow path to the needle lumen via the laser cut holes in the side of the needle. The Luer needle had o-ring inserts into the Luer end to reduce the dead volume of the assembly.

A length of 15 mm by approximately 0.2 mm diameter of an active agent containing composition in the form of an elongated body or filament was loaded from the proximal end into the needle lumen so that it was not blocking the laser cut holes in the side of the needle. The filament comprised 85.6 wt % of 9 micron PLGA microspheres containing 24.5 wt % difluprednate, bound together by 13.2 wt % polyvinylpyrrolidone K90 and 1.2 wt % polyvinylpyrrolidone K12. A 1 mm thick by 4.5 mm diameter section of 60 shore A silicone was placed against the proximal end of the needle which was approximately coplanar with the rear face of the needle hub. The external face of the needle hub was 6.35 mm in diameter and had a 1/4-28 UNF thread cut 12 mm from the proximal end. This thread engaged with a mating thread on an adaptor (female thread to female Luer adaptor) to compress the silicone seal, forming a gas tight annular seal on the needle hub. The Luer adaptor was attached to the drive syringe with the push rod pierced through the silicone seal to form a gas tight seal around the push rod preventing pressure loss out of the needle hub. The needle on the distal end of the device was a 27 gauge thin walled hypodermic needle with a 15 degree beveled tip. A deflecting feature was created in the wall of the needle by mechanically deforming the wall of the tube opposite the bevel using a circular punch. The deflecting feature was located 0.63 mm from the distal tip and had a height inside the needle lumen of 0.11 mm. A distal seal and tissue interface was molded from silicone elastomer of 50A durometer. The seal was 3.0 mm long and had an outer diameter of 0.76 mm and an inner lumen of 0.30 mm diameter. The distal end of the seal was solid in order to provide a seal against the passage of the gas and the material for administration after activation of the device. The distal seal was placed on the distal end of the needle to complete sealing of the gas flow path. The entire gas reservoir and flow path had a dead volume of approximately 20pl in addition to the volume in the gas syringe.

A cadaver porcine eye was prepared by inflating the posterior chamber to a pressure of approximately 20 mm Hg. A target administration location 5 mm posterior of the limbus of the eye was chosen for administration. The activation latch was removed from the gas plunger rod which allowed the gas spring to extend until the internal air compressed to reach an equilibrium position where the force exerted from the gas pressure on the plunger seal and the seal friction was equal to that of the gas spring. The distal seal was placed against the scleral surface and the needle tip was then advanced through the distal seal and into the tissues with the needle bevel oriented towards the posterior of the eye. Once the needle lumen pierced through the distal seal and outer eye tissues, the compressed air entered the suprachoroidal space via the needle lumen and the expansion of the air volume caused the air pressure to drop and allowed the gas plunger rod to advance. The translation of the gas plunger rod allowed the cantilevered spring to extend away from the push rod. With the cantilevered spring no longer blocking the push rod holder the drive spring was able to extend at a reduced speed due to the rotary damper and delivered the solid active agent containing composition over a duration of approximately one second. This functionality ensured that a substantial volume of the compressed air entered the eye before the solid element of drug began to be ejected. The delivery of the solid element was confirmed by manually excising a flap in the sclera to expose the suprachoroidal space. A sample of the fluid in the suprachoroidal space was taken and placed on a microscope slide. Examination of the slide under the microscope at 100X magnification revealed numerous microspheres that had been released from the filament administered into the suprachoroidal space.

## Claims

1. A delivery device comprising
an elongated body (54) with a needle (8) with a lumen at a distal end;
a first reservoir (51) for a volume of gas to be delivered through the needle;
a second reservoir for an active agent containing composition (12) to be delivered through the needle;
a gas flow path from the first reservoir to the needle lumen;
a first plunger (3) with a force element (5) configured to pressurise the volume of gas in the first reservoir prior to or simultaneous with contact of the distal end of the device to a tissue surface;
a second plunger or a push rod (7) configured to provide a delivery force from the force element to the active agent containing composition;
a distal element attached to the distal end of the device configured to seal the needle lumen from delivery of the gas under the delivery force; and
a mechanism configured to provide a sequential delivery process for the volume of gas and the active agent containing composition; wherein:
the distal element comprises a tissue interface and a distal seal (11), and the distal seal is penetrable by a distal tip of the needle by the application of pressure on a tissue surface with the distal end of the device;
the distal element being penetrated is configured to be slidable on the needle to allow advancement of the needle into tissue;
the distal seal being penetrated is configured to open a path for flow or delivery of the gas from the first reservoir through the gas flow path to the distal end of the needle; and
the active agent containing composition from the second reservoir is delivered through the needle by the second plunger or push rod and force element after all or a portion of the volume of gas has been delivered through the needle.

2. The device of claim 1 wherein the force element comprises a first force element configured to apply a delivery force to compress the volume of gas and a second force element configured to apply a delivery force to the active agent containing composition.

3. The device of claim 1 or 2 where the gas flow path comprises the needle lumen, a portion of the needle lumen or a separate lumen.

4. The device of claim 3 where the gas flow path comprises a sleeve coaxial to the needle and a hole in a shaft of the needle within the sleeve.

5. The device of claims 1 to 4 wherein the delivery of the active agent containing composition can be manually triggered by the user after delivery of at least a portion of the volume of gas.

6. The device of claims 1 to 4 wherein the mechanism is configured to automatically trigger the delivery of the active agent containing composition after delivery of at least a portion of the volume of gas.

7. The device of claim 6 wherein the mechanism is configured to sequentially provide a delivery force to the volume of gas and the active agent containing composition.

8. The device of claim 6 wherein the mechanism is configured to block the delivery of the active agent containing composition until delivery of at least a portion of the volume of gas.

9. The device of claim 6, when dependent on claim 2, wherein the mechanism comprises a linkage between the first force element acting on the volume of gas and the second force element acting on the active agent containing composition.

10. The device of claim 6 wherein the mechanism comprises a linkage between the plunger acting on the volume of gas and the plunger or push rod acting on the active agent containing composition.

11. The device of any preceding claim wherein the needle comprises a curved distal tip to direct the active agent containing composition at an angle from the long axis of the needle.

12. The device of any preceding claim wherein the needle comprises an inner deflecting element in the lumen of the needle at the needle bevel to direct the active agent containing composition at an angle from the long axis of the needle.

13. The device of any preceding claim wherein the needle comprises a deflecting element in the lumen of the needle at the needle bevel to fragment the active agent containing composition prior to delivery of the material for administration from the needle distal tip.

14. The device of claim 13 where the deflecting element has a width of 0.20% to 50% of the needle inner diameter.

15. The device of claim 13 where the deflecting element has a height of 0.25% to 50% of the needle inner diameter.

16. The device of any preceding claim additionally comprising a further force element between the body of the device and the distal element configured to provide a forward directed force on the distal element during penetration of the distal seal by the distal tip of the needle.

17. The device of claim 16 wherein the tissue interface comprises an elastomer with a hardness of 10 to 30 Shore A.

18. The device of claim 16 where the forward directed force is in the range of 40 to 82 gram force.

19. The device of any preceding claim further comprising an active agent containing composition wherein the active agent containing composition is a solid or semisolid.

20. The delivery device of any preceding claim wherein the reservoir is within the lumen of the needle.

21. The delivery device of any preceding claim wherein the reservoir is within both the lumen of the needle and an extension of the needle into the body of the device.

22. The device of any preceding claim additionally comprising a collapsible element between the body of the device and the distal element configured to prevent distal movement of the distal element due to the delivery force.

23. The device of claim 22 wherein the collapsible element comprises elongated struts.

24. The device of claim 22 or 23 wherein the collapsible element comprises nitinol or polyimide.

25. The device of any preceding claim additionally comprising a collapsible element between the body of the device and the distal element configured to provide a forward directed force on the distal element during penetration of the distal seal by the distal tip of the needle.

26. The delivery device of claim 25 wherein the collapsible element is configured to provide a constant force after an initial force wherein the initial force is applied during the first 0.5 mm of travel of the distal element proximally along the needle.

27. The device of any preceding claim wherein the tissue interface and distal seal are mounted on a tubular distal housing.

28. The device of claim 27 additionally comprising an elastomeric element which is compressed between the housing and the needle to seal the housing.

29. The delivery device of any one of claims 2 to 28 wherein the first and/or second force element is a spring.

30. The delivery device of any one of claims to 2 to 29 wherein the first and/or second force element is a pressurized gas.

31. The delivery device of any one of claims 2 to 30 wherein the delivery force is activated by a mechanism to compress the first and/or second force element from the exterior of the device.

32. The delivery device of any one of claims 2 to 31 wherein the first and/or second force element is constrained prior to use and the delivery force is activated by mechanically releasing the constrained force element.

33. The delivery device of any preceding claim for administration of an active agent containing composition to the suprachoroidal space or supraciliary space with an effective full needle length of 1 to 4 mm.

34. The delivery device of any one of claims 1 to 32 for administration of an active agent containing composition to the subconjunctival space with an effective full needle length of 0.35 to 2 mm.

## Patentansprüche

1. Abgabevorrichtung, umfassend
einen länglichen Körper (54) mit einer Nadel (8) mit einem Lumen an einem distalen Ende;
ein erstes Reservoir (51) für ein Gasvolumen, das durch die Nadel abgegeben werden soll;
ein zweites Reservoir für eine Wirkstoff enthaltende Zusammensetzung (12), die durch die Nadel abgegeben werden soll;
einen Gasströmungsweg vom ersten Reservoir zum Nadellumen;
einen ersten Kolben (3) mit einem Kraftelement (5), das dazu ausgelegt ist, das Gasvolumen im ersten Reservoir vor oder gleichzeitig mit Kontakt des distalen Endes der Vorrichtung mit einer Gewebeoberfläche mit Druck zu beaufschlagen;
einen zweiten Kolben oder eine Schubstange (7), der bzw. die dazu ausgelegt ist, eine Abgabekraft vom Kraftelement auf die Wirkstoff enthaltende Zusammensetzung bereitzustellen;
ein distales Element, das am distalen Ende der Vorrichtung befestigt ist und dazu ausgelegt ist, das Nadellumen gegen Abgabe des Gases unter der Abgabekraft abzudichten; und
einen Mechanismus, der dazu ausgelegt ist, ein sequenzielles Abgabeverfahren für das Gasvolumen und die Wirkstoff enthaltende Zusammensetzung bereitzustellen; wobei:
das distale Element eine Gewebeschnittstelle und eine distale Dichtung (11) umfasst und die distale Dichtung von einer distalen Spitze der Nadel durch Aufbringen von Druck auf eine Gewebeoberfläche mit dem distalen Ende der Vorrichtung penetrierbar ist;
das distale Element, das penetriert wird, dazu ausgelegt ist, auf der Nadel verschiebbar zu sein, um Vorschieben der Nadel in Gewebe zu ermöglichen;
die distale Dichtung, die penetriert wird, dazu ausgelegt ist, einen Weg für Strömung oder Abgabe des Gases aus dem ersten Reservoir durch den Gasströmungsweg zum distalen Ende der Nadel zu eröffnen; und
die Wirkstoff enthaltende Zusammensetzung aus dem zweiten Reservoir durch die Nadel vom zweiten Kolben oder von der Schubstange und dem Kraftelement abgegeben wird, nachdem das gesamte Gasvolumen, oder ein Teil davon, durch die Nadel abgegeben wurde.

2. Vorrichtung nach Anspruch 1, wobei das Kraftelement ein erstes Kraftelement, das dazu ausgelegt ist, eine Abgabekraft zum Komprimieren des Gasvolumens aufzubringen, und ein zweites Kraftelement, das dazu ausgelegt ist, eine Abgabekraft auf die Wirkstoff enthaltende Zusammensetzung aufzubringen, umfasst.

3. Vorrichtung nach Anspruch 1 oder 2, wobei der Gasströmungsweg das Nadellumen, einen Teil des Nadellumens oder ein separates Lumen umfasst.

4. Vorrichtung nach Anspruch 3, wobei der Gasströmungsweg eine zur Nadel koaxiale Hülse und ein Loch in einem Schaft der Nadel innerhalb der Hülse umfasst.

5. Vorrichtung nach den Ansprüchen 1 bis 4, wobei die Abgabe der Wirkstoff enthaltenden Zusammensetzung vom Benutzer nach Abgabe mindestens eines Teils des Gasvolumens manuell ausgelöst werden kann.

6. Vorrichtung nach den Ansprüchen 1 bis 4, wobei der Mechanismus dazu ausgelegt ist, die Abgabe der Wirkstoff enthaltenden Zusammensetzung nach Abgabe mindestens eines Teils des Gasvolumens automatisch auszulösen.

7. Vorrichtung nach Anspruch 6, wobei der Mechanismus dazu ausgelegt ist, sequenziell eine Abgabekraft auf das Gasvolumen und die Wirkstoff enthaltende Zusammensetzung bereitzustellen.

8. Vorrichtung nach Anspruch 6, wobei der Mechanismus dazu ausgelegt ist, die Abgabe der Wirkstoff enthaltenden Zusammensetzung zu blockieren, bis mindestens ein Teil des Gasvolumens abgegeben wurde.

9. Vorrichtung nach Anspruch 6, in Abhängigkeit von Anspruch 2, wobei der Mechanismus eine Verbindung zwischen dem ersten Kraftelement, das auf das Gasvolumen einwirkt, und dem zweiten Kraftelement, das auf die Wirkstoff enthaltende Zusammensetzung einwirkt, umfasst.

10. Vorrichtung nach Anspruch 6, wobei der Mechanismus eine Verbindung zwischen dem Kolben, der auf das Gasvolumen einwirkt, und dem Kolben oder der Schubstange, der bzw. die auf die Wirkstoff enthaltende Zusammensetzung einwirkt, umfasst.

11. Vorrichtung nach einem der vorhergehenden Ansprüche, wobei die Nadel eine gebogene distale Spitze umfasst, um die Wirkstoff enthaltenden Zusammensetzung in einem Winkel von der langen Achse der Nadel zu leiten.

12. Vorrichtung nach einem der vorhergehenden Ansprüche, wobei die Nadel ein inneres ablenkendes Element im Lumen der Nadel an der Nadelabschrägung umfasst, um die Wirkstoff enthaltenden Zusammensetzung in einem Winkel von der langen Achse der Nadel zu leiten.

13. Vorrichtung nach einem der vorhergehenden Ansprüche, wobei die Nadel ein ablenkendes Element im Lumen der Nadel an der Nadelabschrägung umfasst, um die Wirkstoff enthaltenden Zusammensetzung vor Abgabe des Materials zur Verabreichung aus der distalen Nadelspitze zu fragmentieren.

14. Vorrichtung nach Anspruch 13, wobei das ablenkende Element eine Breite von 0,20% bis 50% des Nadelinnendurchmessers aufweist.

15. Vorrichtung nach Anspruch 13, wobei das ablenkende Element eine Höhe von 0,25% bis 50% des Nadelinnendurchmessers aufweist.

16. Vorrichtung nach einem der vorhergehenden Ansprüche, zusätzlich umfassend ein weiteres Kraftelement zwischen dem Körper der Vorrichtung und dem distalen Element, das dazu ausgelegt ist, eine nach vorne gerichtete Kraft auf das distale Element während der Penetration der distalen Dichtung durch die distale Spitze der Nadel bereitzustellen.

17. Vorrichtung nach Anspruch 16, wobei die Gewebeschnittstelle ein Elastomer mit einer Härte von 10 bis 30 Shore A umfasst.

18. Vorrichtung nach Anspruch 16, wobei die nach vorne gerichtete Kraft im Bereich von 40 bis 82 Gramm Kraft liegt.

19. Vorrichtung nach einem der vorhergehenden Ansprüche, ferner umfassend eine Wirkstoff enthaltende Zusammensetzung, wobei die Wirkstoff enthaltende Zusammensetzung ein Feststoff oder Halbfeststoff ist.

20. Abgabevorrichtung nach einem der vorhergehenden Ansprüche, wobei das Reservoir sich im Lumen der Nadel befindet.

21. Abgabevorrichtung nach einem der vorhergehenden Ansprüche, wobei das Reservoir sich sowohl im Lumen der Nadel als auch in einer Verlängerung der Nadel in den Körper der Vorrichtung befindet.

22. Vorrichtung nach einem der vorhergehenden Ansprüche, zusätzlich umfassend ein kollabierbares Element zwischen dem Körper der Vorrichtung und dem distalen Element, das dazu ausgelegt ist, eine distale Bewegung des distalen Elements aufgrund der Abgabekraft zu verhindern.

23. Vorrichtung nach Anspruch 22, wobei das kollabierbare Element längliche Streben umfasst.

24. Vorrichtung nach Anspruch 22 oder 23, wobei das kollabierbare Element Nitinol oder Polyimid umfasst.

25. Vorrichtung nach einem der vorhergehenden Ansprüche, zusätzlich umfassend ein kollabierbares Element zwischen dem Körper der Vorrichtung und dem distalen Element, das dazu ausgelegt ist, eine nach vorne gerichtete Kraft auf das distale Element während der Penetration der distalen Dichtung durch die distale Spitze der Nadel bereitzustellen.

26. Abgabevorrichtung nach Anspruch 25, wobei das kollabierbare Element dazu ausgelegt ist, eine konstante Kraft nach einer Anfangskraft bereitzustellen, wobei die Anfangskraft während der ersten 0,5 mm der Bewegung des distalen Elements proximal entlang der Nadel aufgebracht wird.

27. Vorrichtung nach einem der vorhergehenden Ansprüche, wobei die Gewebeschnittstelle und die distale Dichtung auf einem röhrenförmigen distalen Gehäuse angebracht sind.

28. Vorrichtung nach Anspruch 27, zusätzlich umfassend ein elastomeres Element, das zwischen dem Gehäuse und der Nadel zum Abdichten des Gehäuses komprimiert wird.

29. Abgabevorrichtung nach einem der Ansprüche 2 bis 28, wobei das erste und/oder zweite Kraftelement eine Feder ist.

30. Abgabevorrichtung nach einem der Ansprüche 2 bis 29, wobei das erste und/oder zweite Kraftelement ein unter Druck stehendes Gas ist.

31. Abgabevorrichtung nach einem der Ansprüche 2 bis 30, wobei die Abgabekraft durch einen Mechanismus aktiviert wird, um das erste und/oder zweite Kraftelement von der Außenseite der Vorrichtung zu komprimieren.

32. Abgabevorrichtung nach einem der Ansprüche 2 bis 31, wobei das erste und/oder zweite Kraftelement vor der Benutzung eingezwängt wird und die Abgabekraft durch mechanische Freigabe des eingezwängten Kraftelements aktiviert wird.

33. Abgabevorrichtung nach einem der vorhergehenden Ansprüche zur Verabreichung einer Wirkstoff enthaltenden Zusammensetzung an den suprachoroidalen Raum oder supraziliären Raum mit einer effektiven vollen Nadellänge von 1 bis 4 mm.

34. Abgabevorrichtung nach einem der Ansprüche 1 bis 32 zur Verabreichung einer Wirkstoff enthaltenden Zusammensetzung an den subkonjunktivalen Raum mit einer effektiven vollen Nadellänge von 0,35 bis 2 mm.

## Revendications

1. Dispositif d'administration comprenant :
un corps allongé (54) avec une aiguille (8), avec une lumière à une extrémité distale ;
un premier réservoir (51) pour un volume de gaz à administrer à travers l'aiguille ;
un second réservoir pour une composition contenant un agent actif (12) à administrer à travers l'aiguille ;
un chemin d'écoulement de gaz du premier réservoir à la lumière de l'aiguille ;
un premier piston (3) avec un élément de force (5) configuré pour mettre sous pression le volume de gaz dans le premier réservoir avant ou simultanément au contact de l'extrémité distale du dispositif avec une surface de tissu ;
un second piston ou une tige de poussée (7) configuré pour fournir une force d'administration de l'élément de force à la composition contenant l'agent actif ;
un élément distal fixé à l'extrémité distale du dispositif configuré pour fermer hermétiquement la lumière de l'aiguille pour empêcher l'administration du gaz sous la force d'administration ; et
un mécanisme configuré pour fournir un processus d'administration séquentiel pour le volume de gaz et la composition contenant un agent actif ;
l'élément distal comprenant une interface tissulaire et un joint distal (11), et le joint distal étant pénétrable par une pointe distale de l'aiguille par l'application d'une pression sur une surface tissulaire avec l'extrémité distale du dispositif ;
l'élément distal en cours de pénétration étant configuré pour pouvoir coulisser sur l'aiguille afin de permettre l'avancement de l'aiguille dans le tissu ;
l'élément distal en cours de pénétration étant configuré pour ouvrir un chemin pour l'écoulement ou l'administration du gaz du premier réservoir à travers le chemin d'écoulement du gaz vers l'extrémité distale de l'aiguille ; et
la composition contenant un agent actif provenant du second réservoir étant administrée à travers l'aiguille par le second piston ou tige de poussée et élément de force après que tout ou partie du volume de gaz a été administré à travers l'aiguille.

2. Dispositif selon la revendication 1, l'élément de force comprenant un premier élément de force configuré pour appliquer une force d'administration pour comprimer le volume de gaz et un second élément de force configuré pour appliquer une force d'administration à la composition contenant un agent actif.

3. Dispositif selon la revendication 1 ou 2, le chemin d'écoulement du gaz comprenant la lumière de l'aiguille, une partie de la lumière de l'aiguille ou une lumière séparée.

4. Dispositif selon la revendication 3, le chemin d'écoulement de gaz comprenant un manchon coaxial à l'aiguille et un trou dans une tige de l'aiguille à l'intérieur du manchon.

5. Dispositif selon les revendications 1 à 4, l'administration de la composition contenant un agent actif pouvant être déclenchée manuellement par l'utilisateur après l'administration d'au moins une partie du volume de gaz.

6. Dispositif selon les revendications 1 à 4, le mécanisme étant configuré pour déclencher automatiquement l'administration de la composition contenant un agent actif après l'administration d'au moins une partie du volume de gaz.

7. Dispositif selon la revendication 6, le mécanisme étant configuré pour fournir séquentiellement une force d'administration au volume de gaz et à la composition contenant un agent actif.

8. Dispositif selon la revendication 6, le mécanisme étant configuré pour bloquer l'administration de la composition contenant un agent actif jusqu'à l'administration d'au moins une partie du volume de gaz.

9. Dispositif selon la revendication 6, lorsqu'elle dépend de la revendication 2, le mécanisme comprenant une liaison entre le premier élément de force agissant sur le volume de gaz et le second élément de force agissant sur la composition contenant un agent actif.

10. Dispositif selon la revendication 6, le mécanisme comprenant une liaison entre le piston agissant sur le volume de gaz et le piston ou la tige de poussée agissant sur la composition contenant un agent actif.

11. Dispositif selon n'importe quelle revendication précédente, l'aiguille comprenant une pointe distale incurvée pour diriger la composition contenant l'agent actif selon un angle par rapport à l'axe long de l'aiguille.

12. Dispositif selon n'importe quelle revendication précédente, l'aiguille comprenant un élément déflecteur interne dans la lumière de l'aiguille au niveau du biseau de l'aiguille pour diriger la composition contenant un agent actif selon un angle par rapport à l'axe long de l'aiguille.

13. Dispositif selon n'importe quelle revendication précédente, l'aiguille comprenant un élément déflecteur dans la lumière de l'aiguille au niveau du biseau de l'aiguille pour fragmenter la composition contenant un agent actif avant l'administration du matériau pour l'administration à partir de la pointe distale de l'aiguille.

14. Dispositif selon la revendication 13, l'élément déflecteur ayant une largeur de 0,20 % à 50 % du diamètre interne de l'aiguille.

15. Dispositif selon la revendication 13, l'élément déflecteur ayant une hauteur de 0,25 % à 50 % du diamètre intérieur de l'aiguille.

16. Dispositif selon n'importe quelle revendication précédente comprenant en outre un autre élément de force entre le corps du dispositif et l'élément distal configuré pour fournir une force dirigée vers l'avant sur l'élément distal pendant la pénétration du joint distal par la pointe distale de l'aiguille.

17. Dispositif selon la revendication 16, l'interface tissulaire comprenant un élastomère ayant une dureté de 10 à 30 Shore A.

18. Dispositif selon la revendication 16, la force dirigée vers l'avant étant comprise entre 40 et 82 grammes de force.

19. Dispositif selon n'importe quelle revendication précédente comprenant en outre une composition contenant un agent actif, la composition contenant un agent actif étant un solide ou un semi-solide.

20. Dispositif d'administration selon n'importe quelle revendication précédente, le réservoir étant à l'intérieur de la lumière de l'aiguille.

21. Dispositif d'administration selon n'importe quelle revendication précédente, le réservoir étant à la fois dans la lumière de l'aiguille et dans une extension de l'aiguille dans le corps du dispositif.

22. Dispositif selon n'importe quelle revendication précédente comprenant en outre un élément pliable entre le corps du dispositif et l'élément distal configuré pour empêcher le mouvement distal de l'élément distal dû à la force d'administration.

23. Dispositif selon la revendication 22, l'élément pliable comprenant des entretoises allongées.

24. Dispositif selon la revendication 22 ou 23, l'élément pliable comprenant du nitinol ou du polyimide.

25. Dispositif selon n'importe quelle revendication précédente comprenant en outre un élément pliable entre le corps du dispositif et l'élément distal configuré pour fournir une force dirigée vers l'avant sur l'élément distal pendant la pénétration du joint distal par la pointe distale de l'aiguille.

26. Dispositif d'administration selon la revendication 25, l'élément pliable étant configuré pour fournir une force constante après une force initiale, la force initiale étant appliquée pendant les premiers 0,5 mm de déplacement de l'élément distal de manière proximale le long de l'aiguille.

27. Dispositif selon n'importe quelle revendication précédente, l'interface tissulaire et le joint distal étant montés sur un logement distal tubulaire.

28. Dispositif selon la revendication 27, comprenant en outre un élément élastomère qui est comprimé entre le logement et l'aiguille pour sceller le logement.

29. Dispositif d'administration selon l'une quelconque des revendications 2 à 28, le premier et/ou le second élément de force étant un ressort.

30. Dispositif d'administration selon l'une quelconque des revendications 2 à 29, le premier et/ou le second élément de force étant un gaz sous pression.

31. Dispositif d'administration selon l'une quelconque des revendications 2 à 30, la force d'administration étant activée par un mécanisme pour comprimer le premier et/ou le second élément de force depuis l'extérieur du dispositif.

32. Dispositif d'administration selon l'une quelconque des revendications 2 à 31, le premier et/ou le second élément de force étant contraint avant l'utilisation et la force d'administration étant activée en libérant mécaniquement l'élément de force contraint.

33. Dispositif d'administration selon n'importe quelle revendication précédente pour l'administration d'une composition contenant un agent actif dans l'espace suprachoroïdien ou l'espace supraciliaire avec une longueur d'aiguille complète effective de 1 à 4 mm.

34. Dispositif d'administration selon l'une quelconque des revendications 1 à 32 pour l'administration d'un agent actif contenant une composition dans l'espace sousconjonctival avec une longueur d'aiguille totale effective de 0,35 à 2 mm.
